# EUROPEAN PATENT SPECIFICATION

(11) **EP 3 930 812 B1**
(45) Date of publication and mention of the grant of the patent: **08.01.2025**
(21) Application number: 20766264.4
(22) Date of filing: 28.02.2020
(51) Int. Cl.: A61M 16/06

(54) **NASAL MASK AND SECUREMENT SYSTEM**
NASALMASKE UND BEFESTIGUNGSSYSTEM
MASQUE NASAL ET SYSTÈME DE FIXATION

(30) Priority: 01.03.2019 US 201962812823 P
(43) Date of publication of application: 05.01.2022
(73) Proprietor: Fisher & Paykel Healthcare Limited, East Tamaki Auckland 2013 (NZ)
(72) Inventor: MICHELSEN, Larissa Grace, Auckland, 2013 (NZ); MEECH, Julio Derek, Auckland, 2013 (NZ); HOWARTH, Brad Michael, Auckland, 2013 (NZ); RONAYNE, Michael Paul, Auckland, 2013 (NZ)
(74) Representative: Forresters IP LLP
(86) International application number: PCT/IB2020/051704
(87) International publication number: WO 2020/178686

(56) References cited:
- WO-A1-2008/011682
- WO-A1-2014/097067
- WO-A1-2015/057083
- WO-A1-2018/042355
- AU-A4- 2005 100 738
- AU-A4- 2005 100 738
- US-A1- 2006 060 200
- US-A1- 2007 163 600
- US-A1- 2014 166 019
- US-A1- 2016 121 068
- US-A1- 2017 348 500
- US-A1- 2017 348 500
- US-A1- 2018 353 721
- US-B2- 9 649 463

## Description

### BACKGROUND

### Field

The present disclosure generally relates to components for medical systems for conveying gases to and/or from a patient. In one particular aspect, the disclosure relates to patient interfaces that form a part of a breathing system.

### Description of the Related Art

In assisted breathing, respiratory gases are supplied to a patient through a patient interface via flexible breathing tube. The patient interface can be a nasal cannula, nasal mask, full face, oral mask or oro-nasal mask, endotracheal tube, or other known types of interfaces. The gases expired by the patient may be channeled through a similar breathing tube to other equipment (valves, ventilators, pressure devices, or the like) or expelled to the patient's surroundings.

In medical applications, such as assisted breathing, the gases inhaled by a patient are preferably delivered close to body temperature (usually between 33°C and 37°C) and with a high relative humidity (commonly near saturation). In other medical applications, such as continuous positive airway pressure (CPAP) systems or positive pressure ventilations systems that provide patient's suffering obstructive sleep apnea (OSA) with positive pressure breathing gases, the breathing gases may be heated and/or humidified to varying levels to improve user comfort or supplied without heating or humidification.

In the specification where reference has been made to patent specifications, other external documents, or other sources of information, this is generally for the purpose of providing a context for discussing the features of the disclosure. Unless specifically stated otherwise, reference to such external documents is not to be construed as an admission that such documents, or such sources of information, in any jurisdiction, are prior art, or form part of the common general knowledge in the art.
US2017348500 relates to a patient interface. US2007163600 relates to a user interface and head gear for a continuous positive airway pressure device. US2016121068 relates to a patient interface device with limited support area on the face. WO2018042355 relates to a patient interface, system and method. US2018353721 relates to patient interface systems. AU2005100738 relates to a patient interface device. US2014166019 relates to a headgear apparatus for a nasal interface. US2006060200 relates to a cheek mounted patient interface.

### SUMMARY

The invention is defined in the appended claims. It would be advantageous to provide a system for an alternative or improved interface location or operational positioning of the interface, such as a nasal cannula, nasal mask, or oro-nasal mask. Such an alternative or improved system may further assist with improved compliance of gas delivery treatment.

The claimed invention provides a patient interface as claimed.

An aspect of the disclosure involves a patient interface comprising a mask seal body defining a breathing chamber and having a user-contacting surface configured to contact a face of the user and surround at least the nares of the user. The interface also comprises a pair of facial pads, each of the facial pads configured to engage the face of a user on opposing sides of the mask seal body. The interface further comprises a pair of bridging portions, each of the bridging portions connecting the mask seal body and a respective one of the facial pads. Each of the bridging portions has a curved shape configured in use to urge the mask seal body toward the user's face to create or maintain sealing engagement between the user-contacting surface and the user's face.

In some configurations, the bridging portion comprises one or more cutouts.

In some configurations, a mechanical fastener component is positioned within the cutout(s).

In some configurations, the mechanical fastener component is a hook or loop material.

In some configurations, the bridging portion comprises one or more locating features.

In some configurations, as the one or more locating features are grooves.

In some configurations, a mechanical fastening material, such as a hook or loop material is positioned within the locating feature(s) such as groove(s).

In some configurations, a mechanical fastener component is positioned within the groove(s).

In some configurations, the bridging portion comprises a portion having a mechanical fastener component.

In some configurations, the mechanical fastener component is a hook or loop material.

In some configurations, the bridging portion comprises a clip configured to retain a respiratory tube.

In some configurations, each of the bridging portions is split into an upper section and a lower section that are separate from one another.

In some configurations, the bridging portions are constructed integrally with the mask seal body.

In some configurations, the bridging portions are constructed separately from the mask seal body and connected thereto.

In some configurations, the bridging portion comprises an S-shape, a U-shape or a C-shape as viewed from above.

In some configurations, the patient interface further comprises a reinforcing element configured to support the structure of the mask seal body.

In some configurations, the reinforcing element is an elongated strip overmoulded on or within the mask seal body.

In some configurations, the reinforcing element is a clip positioned at a front surface of the mask seal body.

In some configurations, the bridging portion is integrally constructed with the clip.

In some configurations, the reinforcing element is a hardened interface tube connection attached to the mask seal body.

In some configurations, the reinforcing element is a thickened portion of the mask seal body.

In some configurations, the reinforcing element is at least one of (i) an elongated strip overmoulded on or within the mask seal body; (ii) a clip positioned at a front surface of the mask seal body; (iii) a hardened interface tube connection attached to the mask seal body; and (iv) a thickened portion of the mask seal body.

In some configurations, the bridging portion is integrally constructed with the clip.

In some configurations, the mask seal body comprises an upper region, wherein at least part of the upper region is configured to roll when the mask seal body is positioned on a user's face.

In some configurations, the upper region comprises a portion with a thinner wall located between portions having thicker walls.

In some configurations, the mask seal body is configured to be placed around an alar region of a user.

In some configurations, the mask seal body includes an under-nose support configured to be placed under a nose of a user when the interface is worn by the user.

In some configurations, the interface includes one or more respiratory tubes configured to deliver gas.

In some configurations, the respiratory tubes comprise an inspiratory tube configured to deliver gas to a user.

In some configurations, the respiratory tubes comprise an expiratory tube configured to deliver gas from a user.

In some configurations, the mask seal body includes a tube receiving portion configured to receive the one or more respiratory tubes.

In some configurations, the tube receiving portion comprises elongate tube extensions extending from the mask seal body.

In some configurations, the elongate tube extensions vary in cross-sectional size or shape along a length of the elongate tube extensions.

In some configurations, the elongate tube extensions further comprise regions with a first part of a releasable connector for connection with a second part of a releasable connector on the respective bridging portion.

In some configurations, the bridging portion is configured to receive the respiratory tube.

In some configurations, the tube receiving portion comprises one or more swivel connectors.

In some configurations, the mask is overmolded to the respiratory tubes.

In some configurations, the one or more swivel connectors comprise elbows having an angle of less than 90 degrees, preferably about 45 degrees.

In some configurations, the one or more respiratory tubes connect to the mask seal body at an angle of between 0-90 degrees, preferably about 45 degrees.

In some configurations, at least one of the bridging portions defines an internal flow path.

In some configurations, the patient interface further comprises a patch assembly coupled to each of the facial pads for securing the facial pads to the user.

In some configurations, the patch assembly comprises a first portion coupled to the facial pad and a second portion configured to be coupled to the user, wherein the patch assembly is configured for releasable connection between the first portion and the second portion.

In some configurations, the patient interface further comprises a third facial pad configured to engage the forehead of the user above the mask seal body and a third bridging portion connecting the mask seal body and the third facial pad.

In some configurations, the mask seal body comprises a reduced stiffness region.

In some configurations, the reduced stiffness region is located in an upper portion of the mask seal body.

In some configurations, the reduced stiffness region comprises a reduced thickness relative to portions of the mask seal body above and/or below the reduced stiffness region.

In some configurations, the reduced stiffness region has a triangular or substantially triangular profile.

In some configurations, the triangular or substantially triangular profile has a rounded apex.

In some configurations, an upper edge of the reduced stiffness region defines a dip.

In some configurations, the reduced stiffness region is a rolling region configured to roll onto another portion of the mask seal body.

An aspect of the present disclosure, which is not independently claimed as such, but which may be used in combination with the claimed invention, involves a patient interface comprising a mask seal body defining a breathing chamber and having a user-contacting surface configured to contact a face of the user and surround at least the nares of the user. The patient interface also comprises a pair of facial pads, each of the facial pads configured to engage the face of a user on opposing sides of the mask seal body. The facial pads are coupled to the mask seal body.

In some configurations, a sealing mechanism is configured to urge the mask seal body toward the user's face to create or maintain sealing engagement between the user-contacting surface and the user's face.

In some configurations, the sealing mechanism is integral with the mask seal body.

In some configurations, the sealing mechanism is formed separately from the mask seal body.

In some configurations, the sealing mechanism comprises a strap.

In some configurations, the sealing mechanism comprises a bridging portion that extends between the mask seal body and each of the facial pads.

In some configurations, the bridging portion defines a curved shaped configured to roll in response to movement between the facial pad and the mask seal body.

In some configurations, the bridging portion comprises one or more hinges.

In some configurations, the bridging portion comprises one or more cutouts.

In some configurations, a hook or loop material is positioned within the cutout(s).

In some configurations, a mechanical fastener component is positioned within the cutout(s).

In some configurations, the bridging portion comprises one or more locating features, such as grooves.

In some configurations, a hook or loop material is positioned within the locating feature(s) such as groove(s).

In some configurations, a mechanical fastener component is positioned within the groove(s).

In some configurations, the bridging portion comprises a portion having a mechanical fastener component.

In some configurations, the mechanical fastener component is a hook or loop material.

In some configurations, the bridging portion comprises a clip configured to retain a respiratory tube.

In some configurations, each of the bridging portions is split into an upper section and a lower section that are separate from one another.

In some configurations, wherein the patient interface further comprises a reinforcing element configured to support the structure of the mask seal body.

In some configurations, the reinforcing element is an elongated strip overmoulded on or within the mask seal body.

In some configurations, the reinforcing element is a clip positioned at a front surface of the mask seal body.

In some configurations, the bridging portion is integrally constructed with the clip.

In some configurations, the reinforcing element is a hardened interface tube connection attached to the mask seal body.

In some configurations, the reinforcing element is a thickened portion of the mask seal body.

In some configurations, the mask seal body comprises an upper region, wherein at least part of the upper region is configured to roll when the mask seal body is positioned on a user's face.

In some configurations, the upper region comprises a portion with a thinner wall located between portions having thicker walls.

In some configurations, the mask seal body is configured to be placed around an alar region of a user.

In some configurations, the mask seal body includes an under-nose support configured to be placed under a nose of a user when the interface is worn by the user.

In some configurations, the patient interface further comprises one or more respiratory tubes configured to deliver gas.

In some configurations, the respiratory tubes comprise an inspiratory tube configured to deliver gas to a user.

In some configurations, the respiratory tubes comprise an expiratory tube configured to deliver gas from a user.

In some configurations, the mask seal body includes a tube receiving portion configured to receive the one or more respiratory tubes.

In some configurations, the tube receiving portion comprises elongate tube extensions extending from the mask seal body.

In some configurations, the elongate tube extensions vary in cross-sectional size or shape along a length of the elongate tube extensions.

In some configurations, the tube receiving portion comprises one or more swivel connectors.

In some configurations, the one or more swivel connectors comprise elbows having an angle of less than 90 degrees, preferably about 45 degrees.

In some configurations, the one or more respiratory tubes connect to the mask seal body at an angle of between 0-90 degrees, preferably about 45 degrees.

In some configurations, at least one of the bridging portions defines an internal flow path.

In some configurations, the patient interface further comprises a patch coupled to each of the facial pads for securing the facial pads to the user.

In some configurations, the patch comprises a first portion coupled to the facial pad and a second portion configured to be coupled to the user, wherein the patch assembly is configured for releasable connection between the first portion and the second portion.

In some configurations, a third facial pad configured to engage the forehead of the user above the mask seal body and a third bridging portion connecting the mask seal body and the third facial pad.

In some configurations, the mask seal body comprises a reduced stiffness region.

In some configurations, the reduced stiffness region is located in an upper portion of the mask seal body.

In some configurations, the reduced stiffness region comprises a reduced thickness relative to portions of the mask seal body above and/or below the reduced stiffness region.

In some configurations, the reduced stiffness region has a triangular or substantially triangular profile.

In some configurations, the triangular or substantially triangular profile has a rounded apex.

In some configurations, an upper edge of the reduced stiffness region defines a dip.

In some configurations, the reduced stiffness region is a rolling region configured to roll onto another portion of the mask seal body.

Further aspects and advantages of the present disclosure will become apparent from the ensuing description which is given by way of example only.

### BRIEF DESCRIPTION OF THE DRAWINGS

Throughout the drawings, reference numbers can be reused to indicate general correspondence between reference elements. The drawings are provided to illustrate example embodiments described herein and are not intended to limit the scope of the disclosure.
FIG. 1 shows an example of a system in which embodiments of the patient interface can be used.
FIG. 2A is a front, top and side perspective view of a mask seal.
FIG. 2B is a top view of the mask seal of FIG. 2A.
FIG. 2C is a front view of the mask seal of FIG. 2A.
FIG. 2D is a rear view of the mask seal of FIG. 2A.
FIG. 3 is a schematic drawing of a rear view of a mask seal.
FIG. 4 is a schematic drawing showing a region of the face of a user where a mask seal is applied.
FIG. 5 is a rear, top and side perspective view of a mask seal.
FIG. 6A is a front, top and side perspective transparent view of a mask seal revealing the internal structure of the mask seal.
FIG. 6B is a top projected view of the mask seal of FIG. 6A.
FIG. 6C is a front projected view of the mask seal of FIG. 6A.
FIG. 6D is a rear projected view of the mask seal of FIG. 6A.
FIG. 7A is a front, top and side perspective view of a reinforcing structure for a mask seal.
FIG. 7B is a top view of the reinforcing structure of FIG. 7A.
FIG. 7C is a front view of the reinforcing structure of FIG. 7A.
FIG. 8A is a front, top and side perspective view of a mask seal.
FIG. 8B is a side view of the mask seal of FIG. 8A.
FIG. 8C is a front view of the mask seal of FIG. 8A.
FIG. 9A is a front, top, and side perspective view of a patient interface.
FIG. 9B is a top view of the patient interface of FIG. 9A.
FIG. 9C is a front view of the patient interface of FIG. 9A.
FIG. 9D is a rear view of the patient interface of FIG. 9A.
FIG. 10A is a front, top and side perspective view of a mask seal.
FIG. 10B is a top view of the mask seal of FIG. 10A.
FIG. 11 is a top view of a mask seal positioned on a user's face.
FIG. 12A is front, top and side perspective view of a mask seal.
FIG. 12B is a front, top and side perspective projected view of the mask seal of FIG. 12A.
FIG. 12C is a top projected view of the mask seal of FIG. 12A.
FIG. 12D is a front projected view of the mask seal of FIG. 12A.
FIG. 12E is a rear projected view of the mask seal of FIG. 12A.
FIG. 13 is a front, top and side perspective view of a mask seal.
FIG. 14 is a front, top and side perspective view of a mask seal.
FIGS. 15A-B are front, top and side perspective views of a patient interface.
FIG. 16A is a front, top and side perspective view of a mask seal.
FIG. 16B is a top view of the mask seal of FIG. 17A with an interface tube.
FIG. 17A is a perspective view of a connector for a patient interface.
FIG. 17B is another perspective view of the connector of FIG. 18A.
FIG. 18A is a front, top and side perspective view of a mask seal.
FIG. 18B is a top view of the mask seal of FIG. 18A.
FIG. 18C is a front view of the mask seal of FIG. 18A.
FIG. 18D is a rear view of the mask seal of FIG. 18A.
FIG. 19 is a front, top and side perspective view of a mask seal.
FIG. 20 is a front, top and side perspective view of a mask seal.
FIG 21A is a front, top and perspective view of a mask seal.
FIG. 21B is a front view of the mask seal of FIG. 21A.
FIG. 21C is a rear view of the mask seal of FIG. 21A
FIG. 21D is a side view of the mask seal of FIG. 21A.

### Detailed Description

Embodiments of systems, components and methods of assembly and manufacture will now be described with reference to the accompanying figures, wherein like numerals refer to like or similar elements throughout. Although several embodiments, examples and illustrations are disclosed below, it will be understood by those of ordinary skill in the art that the disclosures described herein include other uses of the disclosures and obvious modifications and equivalents thereof. The terminology used in the description presented herein is not intended to be interpreted in any limited or restrictive manner simply because it is being used in conjunction with a detailed description of certain specific embodiments of the disclosures. In addition, embodiments of the disclosures can comprise several novel features and no single feature is solely responsible for its desirable attributes or is essential to practicing the disclosures herein described.

Certain terminology may be used in the following description for the purpose of reference only, and thus are not intended to be limiting. For example, terms such as "above" and "below" refer to directions in the drawings to which reference is made or directions relative to an orientation of the device as used with the user in an upright position. Terms such as "front," "back," "left," "right," "rear," and "side" describe the orientation and/or location of portions of the components or elements within a consistent but arbitrary frame of reference which is made clear by reference to the text and the associated drawings describing the components or elements under discussion or relative to an orientation of the device as used with the user in an upright position.

### Respiratory System

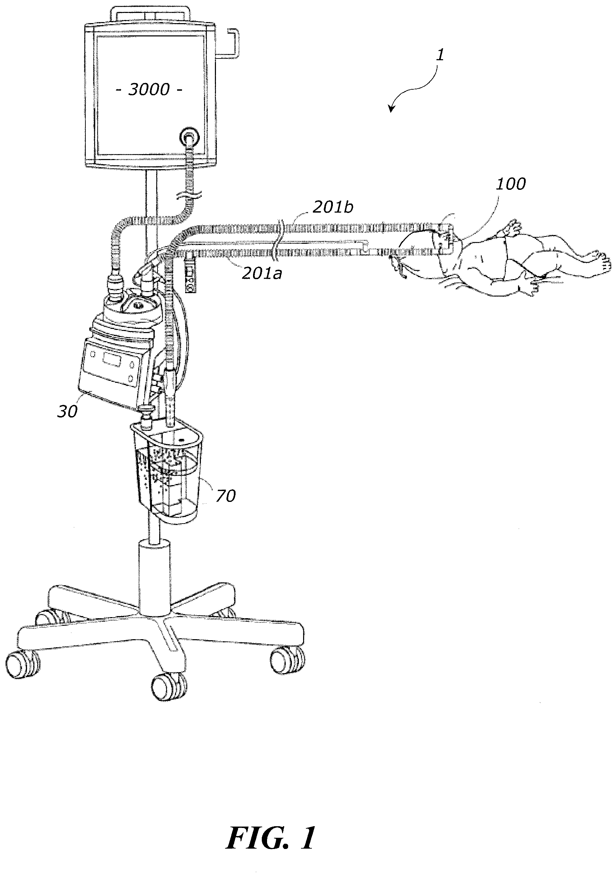

FIG. 1 shows an example respiratory system 1 in which embodiments of the patient interface 100 can be used. In the illustrated arrangement, the patient interface 100 receives an inspiratory flow of gases via an inspiratory conduit 201a, and a flow of the expiratory gases is directed from the interface 100 via an expiratory conduit 201b to a resistance device, which in the illustrated arrangement is a bubbler device 70. An optional humidifier system 30 is provided to humidify the inspiratory flow of gases. The humidifier system 30 typically includes a chamber sitting atop a heater base, the chamber of which is fed with a source of gases flow from, for example, a hospital or other supply source 3000. The humidified inspiratory flow of gases is delivered to the airway of the patient by inspiratory conduit 201a and the patient interface 100. Excess and expired gases are evacuated from the patient interface 100 by the expiratory conduit 201b. The resistance device 70 provides resistance to the expiratory flow of the gases exiting the system 1 to the atmosphere to provide a desirable peak end expiratory pressure (PEEP). One of skill in the art will understand that such a system may include additional and/or replacement components as are known in the art. In some embodiments, the patient interface 100 is a mask. For example, the patient interface 100 may be a nasal mask, an oro-nasal mask, an oral mask or a full-face mask. In some embodiments, the resistance device and/or the humidifier may be integrated into the supply source 3000. Although a water-based resistance device is shown in FIG. 1, it should be appreciated by one of skill that the resistance device may be any other mechanical or electrical resistance device as is known in the art.

### Patient Interface

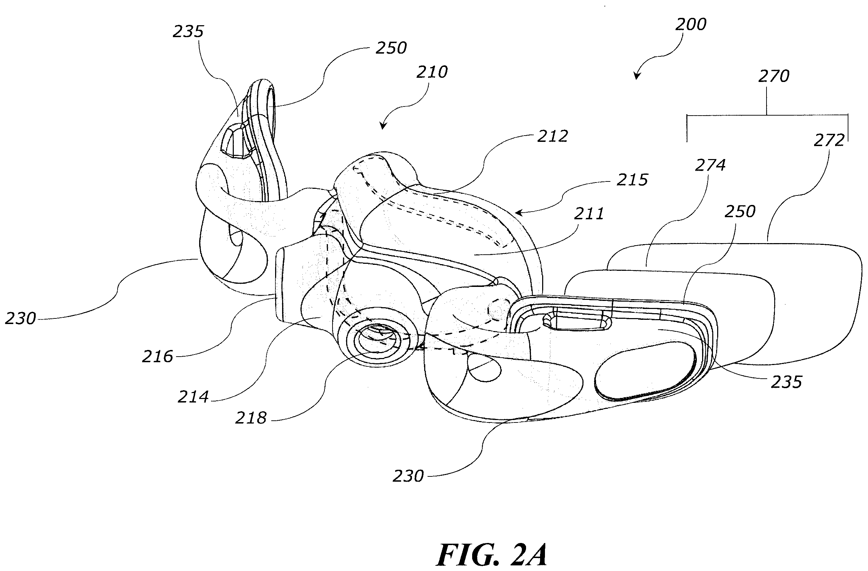

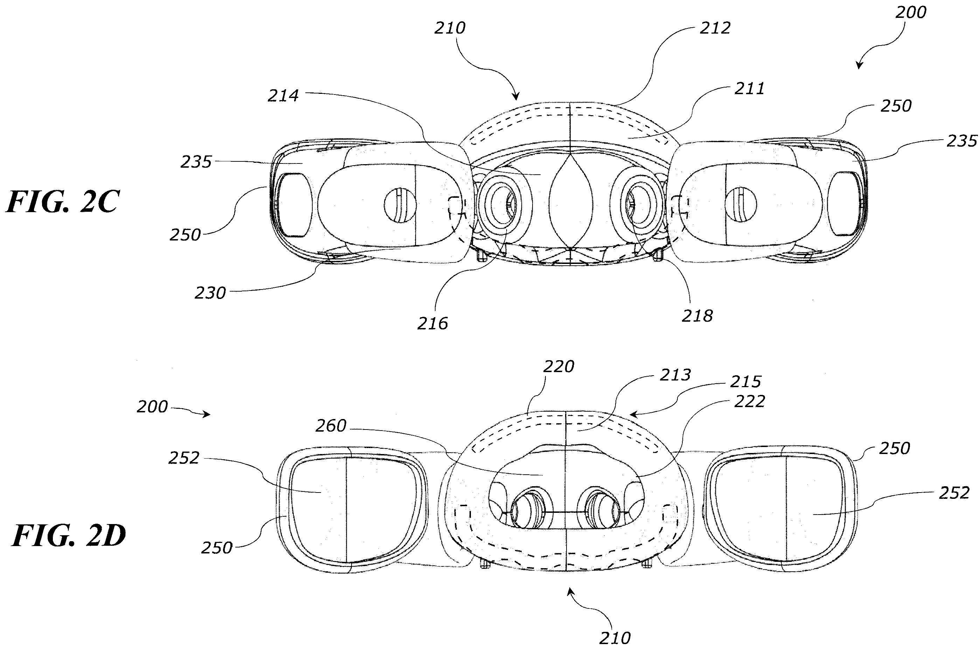

FIGS. 2A-D illustrates a mask 200 which may comprise or be included in a patient interface, such as the patient interface 100. In some embodiments, the mask 200 includes a seal body 210 which may be applied on the face of a patient. In the illustrated arrangement, the seal body 210 is a nasal seal applied over a nose of a patient, forming a seal on or about the nose surrounding the nares of the patient. In some embodiments, the seal body 210 could cover both a nose and a mouth of the patient. The illustrated mask 200 includes one or more feet, cheek supports or facial pads 250 for the securement or supporting of the mask 200 relative to the user, and one or more side arms or bridging portions 230 each extending from the seal body 210 and connecting the facial pad 250 with the seal body 210.

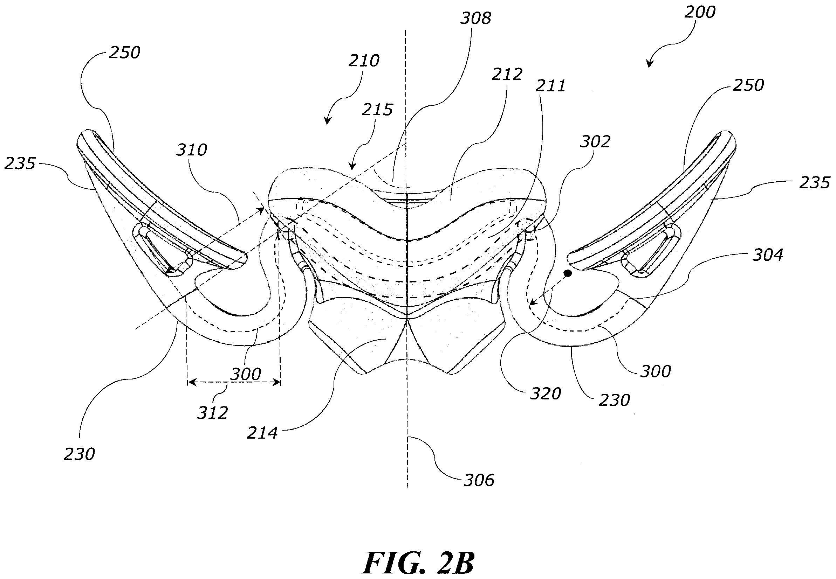

In the illustrated arrangement, each of the facial pads 250 includes a base or connecting portion 235 that is coupled directly to the respective bridging portion 230. In some configurations, the base or connecting portion 235 is configured to provide support to a face-contacting portion of the facial pad 250. For example, the base or connecting portion 235 can assist in maintaining a shape (e.g., a curved shape) of the face-contacting portion of the facial pad 250. In the illustrated arrangement, the base or connecting portion 235 is generally triangular in shape as viewed from above (see, e.g., Fig. 2B) and includes a long strut portion and a short strut portion that cooperate to define an opening that passes through the base or connecting portion 235 in a vertical direction (relative to the orientation in the figures or as worn with the user in an upright position). It should be appreciated by one of skill in the art that such an opening may reduce cost (for example, by reducing material) and impart flexibility on the connection between the bridging portion and the facial pad 250. However, such an opening may be optional as it may add complexity in manufacturing the interface. Therefore, the base or connecting portion 235 may be solid. Alternatively, rather than having a long and short strut portions, the opening may define any suitable shape.

In some embodiments, the seal body 210 is permanently or semi-permanently connected with the bridging portions 230. In some embodiments, the seal body 210 and the bridging portions 230 are integrally formed in a single, unitary piece. In some embodiments, the seal body 210, the bridging portions 230, and the facial pads 250 may be integrally formed in a single, unitary piece. For example, the seal body 210, the bridging portions 230 and the facial pads 250 may be molded as a single piece from a single material, or co-molded as a single piece from different materials. In some embodiments, the seal body 210 and the bridging portions 230 are removably or releasably coupled such that the seal body 210 may be removed for cleaning or replacement if required. Additionally, the ease of manufacturing may be improved if the seal body 210 and the bridging portions 230 are individually formed and subsequently assembled or coupled.

As shown in FIGS. 2A-B, in some embodiments, the seal body 210 includes a tube receiving portion 214 in the form of a tube coupling or manifold for connecting a breathing chamber or mask cavity 260 of the seal body 210 with one or more interface tubes (not shown). In some embodiments, the tube receiving portion 214 may be connected to an inspiratory tube and an expiratory tube. In some embodiments the inspiratory tube and expiratory tube may be coaxial. In some embodiments, the tube receiving portion 214 may be connected directly or indirectly with conduits similar to, for example, the inspiratory conduit 201a and/or the expiratory conduit 201b described in relation to FIG. 1. In some embodiments, as illustrated in FIGS. 2A-B, the tube receiving portion 214 may include openings 216, 218 which can receive the interface tubes and allow gas flow from and to the patient via the mask cavity 260 of the seal body 210. In some embodiments, the patient ends of interface tubes may be overmoulded together or connected to a single piece. In this embodiment, the tube receiving portion 214 may have a single opening to receive the single piece. In some embodiments, a tube receiving portion with a single opening may connect to receive a tube in which the inspiratory and expiratory tube are coaxial. In some embodiments, the seal body 210 includes the tube receiving portion 214 at the anterior side of the seal body 210, or a non-patient facing side of the seal body 210. As illustrated in FIGS. 2A-D, the tube receiving portion 214 is shaped to receive the interface tubes at an angle of approximately 45° relative to the width (side-to-side) direction of the seal body 210. In some embodiments, the tube receiving portions 214 is shaped to receive the interface tubes in a perpendicular direction to the width of the seal body 210, or in a parallel direction to the width of the seal body 210.

The seal body 210 comprises a cushion portion 215 that defines a face-contacting surface 220 having an inner peripheral edge 222 that defines a nose-receiving opening into the mask cavity 260 for receiving the user's nose in use. In some embodiments, the tube receiving portion 214 may be located generally opposite the face-contacting surface 220. In use, the patient interface 100 is secured against the user's face such that the contacting surface 220 of the seal body 210 envelops or circumscribes a portion of the user's nose defining the nares and sealingly engages about the user's nose such as against the cheek surfaces and/or lateral side surfaces of the user's nose, the upper lip region below the user's nose, and across the nasal bridge region or nasal tip region of the user's nose. The seal body 210 may be substantially hollow and generally shaped to provide or define the seal cavity 260 for receiving the flow of gases from the tube receiving portion 214. The seal cavity 260 may be fluidically connected with the openings 216 and 218, such that gas from and to the patient flows through the cavity 260.

The seal body 210 may include an upper portion 212 at the top of the seal body 210. The upper portion 212 may be configured to extend over a nasal bridge and/or nasal tip and/or ala of the user. In some embodiments, the upper portion 212 may be designed to roll over onto an outer surface the seal body 210. In some embodiments, the upper portion 212 may roll onto another portion of the seal body 210. To assist with the rolling of the upper portion 212, the upper portion 212 may have a varying thickness or a varying stiffness. In some embodiments, to induce the upper portion 212 to roll, a reduced stiffness region 211 can be incorporated. The reduced stiffness region 211 can reduce or eliminate the likelihood of the seal body 210 buckling or adversely deforming in a region other than the desired region for rolling. In some embodiments, other means for providing the reduced stiffness region 211 also can be used to induce rolling of the upper portion 212. For example, the material of the upper portion 212 can be configured to have a reduced stiffness through material selection or material properties. In addition, a composite of materials can be used to provide a region of reduced stiffness or rigidity. Moreover, a combination of any suitable techniques can be used.

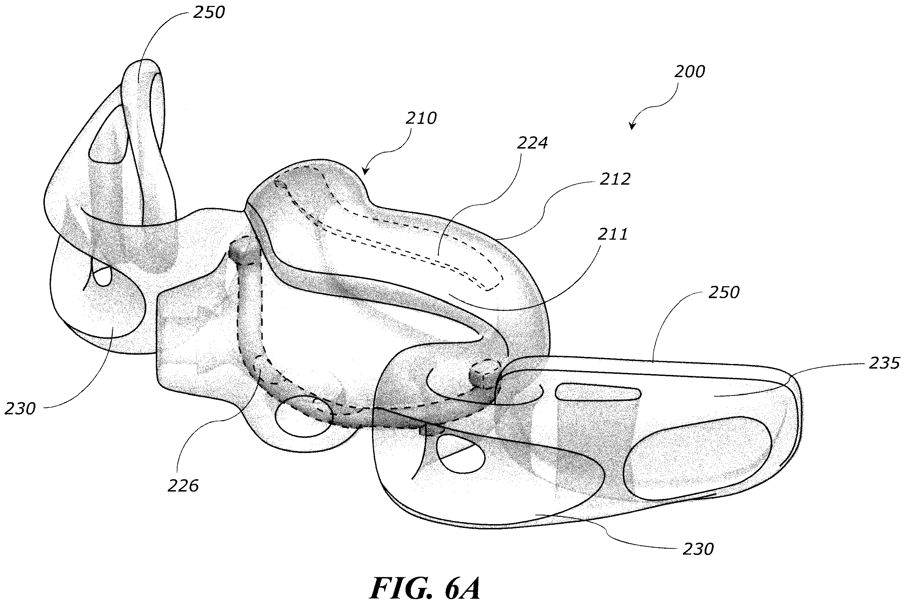

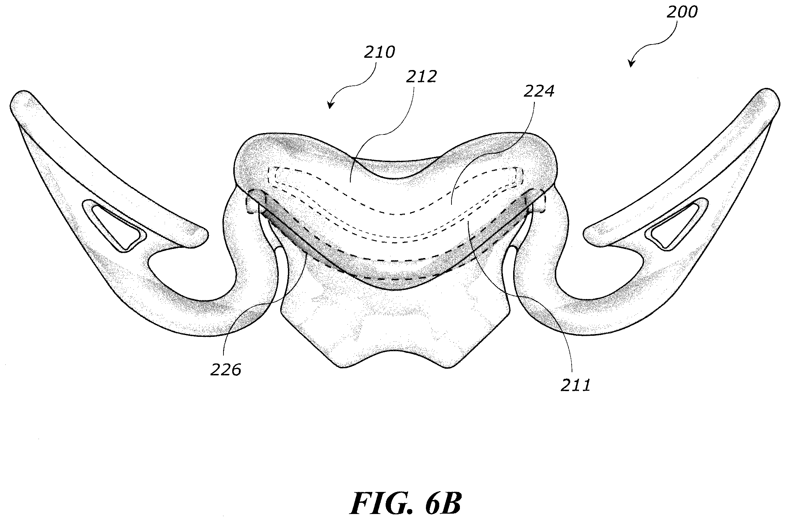

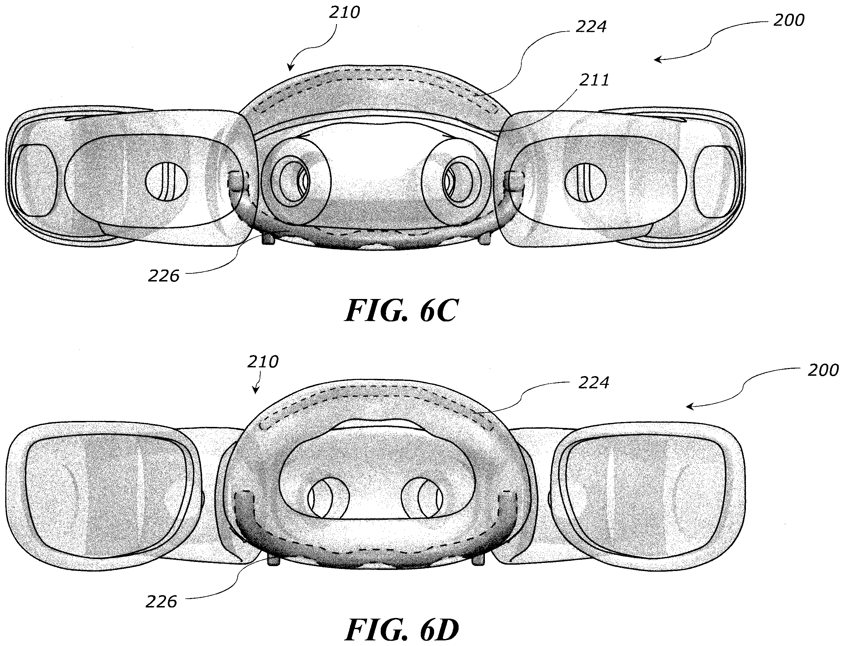

When the upper portion 212 comprises the reduced stiffness region 211, the upper portion 212 of the seal body 210 may tend to balloon outward under internal pressures, such as those encountered during positive pressure therapy regimens, which ballooning is believed to be caused by the reduced stiffness region 211 that defines a large area of silicone or flexible materials without significant structure. With reference to Figures 6A-B, to reduce the prevalence of ballooning in the upper portion 212 and/or to provide enhanced structure in the upper portion 212, a support structure, such as a band 224, can be positioned along at least a portion of the upper portion 212. For example, the band 224 can be formed at least in part by a region of significantly increased thickness relative to the reduced stiffness region 211, where the region is formed of the same material forming the seal body 210. In other arrangements, the band 224 can be or include a component formed of a material that has increased stiffness relative to the silicone or other material(s) forming the seal body 210.

In some embodiments, the band 224 can be a separately formed component that is at least partially encased by the material of the seal body 210. For example, the band 224 can be a preformed plastic component and the seal body 210 can be overmoulded onto or around the band 224. In some configurations, the band 224 can be defined by a portion of the upper portion 212 that has enhanced stiffness relative to surrounding regions. For example, but without limitation, the band 224 can be defined by one or both of a portion of increased thickness, a portion of differing materials or material properties that result in increased stiffness or the like. The band 224 may extend along at least a portion of the upper portion 212 of the seal body 210.

In some embodiments, the face-contacting surface 220 may be shaped such that the face-contacting surface 220 substantially corresponds to or can accommodate the contour of the face of the user and form a fluid-tight seal or a substantially fluid-tight seal during normal use. For example, as illustrated in FIG. 2B, in a top view, the left and right sides of the seal body 210 protrude into the patient's alar region, as opposed to the middle portion, which curves away from the face to accommodate the nose. Moreover, in the illustrated arrangement, the top surface of the seal body 210 (which contacts the nose) dips further away than the lower surface of the seal body 210 (which contacts the upper lip). This allows the seal body 210 to be positioned lower down on the nose of the user in comparison to designs having a flatter face-contacting surface. Advantageously, such an arrangement causes or allows the seal body 210 to be positioned away from the eyes and on a region of the nose made up of cartilage and fat, which is less likely to be susceptible to skin irritation than regions with bone and less fat.

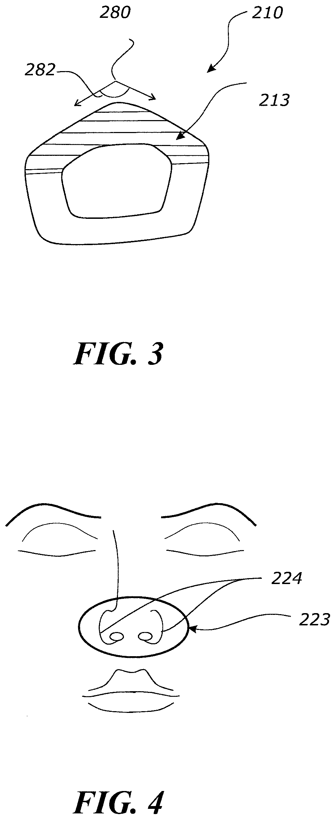

FIG. 3 illustrates a schematic view of the seal body 210 from the face-contacting side or posterior side of the seal body 210. In some embodiments, as shown by a striped region in FIG. 3, a region 213 of the upper portion 212 ("rolling region") may be designed to roll as described herein. The rolling region 213 can be formed by and/or be coextensive with a reduced stiffness region (such as the reduced stiffness region 211 of FIGS. 2A-2D or 6A-6D). In some embodiments, the wall thickness of the seal body 210 may be substantially constant through the rolling region 213, while relatively thicker along the bottom and front portions of the mask, thereby providing support for the structure of the seal body 210. The rolling region 213 may thus comprise a thin walled portion positioned between a pair of comparatively thicker walled portions. The rolling region 213 can be positioned on or adjacent the upper portion 212 of the seal body 210 to permit at least part of the upper portion 212 to deform downwardly relative to a lower portion of the seal body 210. The rolling region 213 may extend laterally across the seal body 210. However, other arrangements to induce or facilitate rolling in the rolling region 213 can also be used. Examples of mask seals comprising rolling portions are disclosed in WO2014/062070.

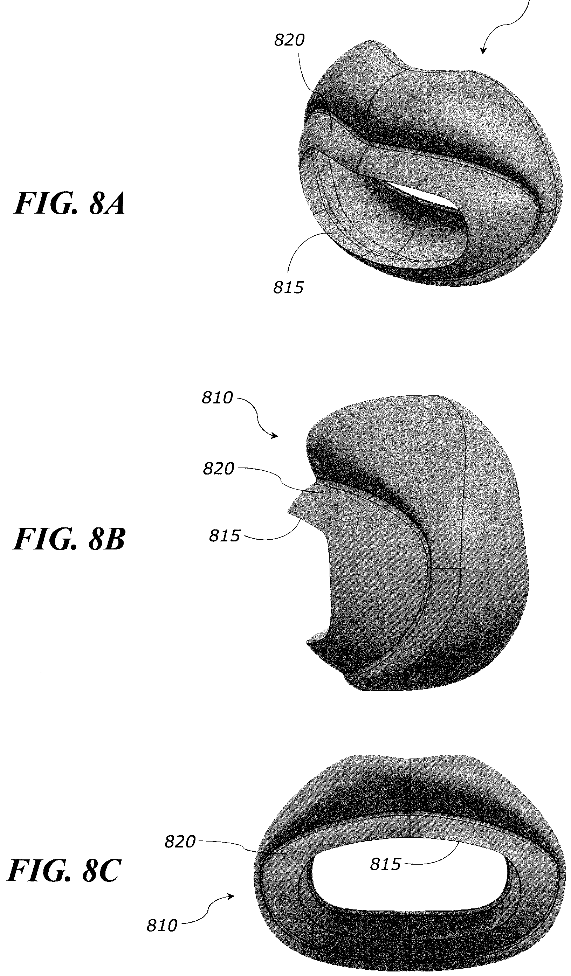

As shown in FIG. 3, the rolling region may have a triangular or substantially triangular profile, with an apex 280. The apex 280 can be defined as a tip, a top and an angular summit of the seal body 210, in which apex 280 is positioned in proximity to the nasal bridge of the user when in use. The apex 280 may have an angle 282 selected to be large enough to provide a relatively flat profile, such that the apex 280 of the seal body 210 does not interfere with eyes of the user. In other arrangements, the apex may be very slight and/or rounded, rather than triangular. In some embodiments, there may be no central or single apex, and instead, a central portion of an upper edge of the seal may dip down, such as in the seal body 810 of FIGS. 8A-C. Such designs may advantageously avoid sealing across the sensitive bridge of the nose, as discussed above.

In some embodiments, the bridging portions 230 may be connected to the seal body 210 at a region other than the rolling region 213 of the upper portion 212 of the seal body 210, such that the movement of the bridging portions 230 do not interfere with the rolling of the rolling region 213. For example, the bridging portions 230 may be connected to a side portion, a bottom portion, or a front portion of the seal body 210.

In some embodiments, the inner peripheral edge 222 of the seal body 210 is shaped and/or sized such that the face-contacting surface 220 does not touch the alar region of the nose of the user. As a result, as shown in FIG. 4, a mask seal region 223, which is an area defined by a location of the inner peripheral edge 222 on the face of the user, extends above the upper lip at its lower end, below the bridge of the nose (i.e. near the supratip of the nose) at its upper end, and away from the alae 224 of the nose on its side. In some embodiments, the seal body 210 may be sized and/or shaped to have different mask seal regions. For example, the seal body 210 may be an oro-nasal mask, and the mask seal region may extend around a user's nose and mouth. In some embodiments, the seal body 210 may include sealing pillows and/or nasal prongs to facilitate sealing with nares of the user. In some embodiments, the seal body 210 may be sized and/or shaped to seal in the alar region of the patient.

In some embodiments, the seal body 210 may include an under-nose support, which can contact or engage under the user's nose to generate an opposing downward force that at least partially counteracts, resists or otherwise mitigates the resultant force generated by the patient interface and exhalation force to thereby stabilize the nasal mask in place over the user's nose during use and/or inhibit or prevent its tendency to slide or otherwise move up the user's face from its initially secured position prior to gas delivery being initiated. An under-nose support may also help to resist rocking of the mask against the patient's face, in particular, vertical or chin to forehead rocking.

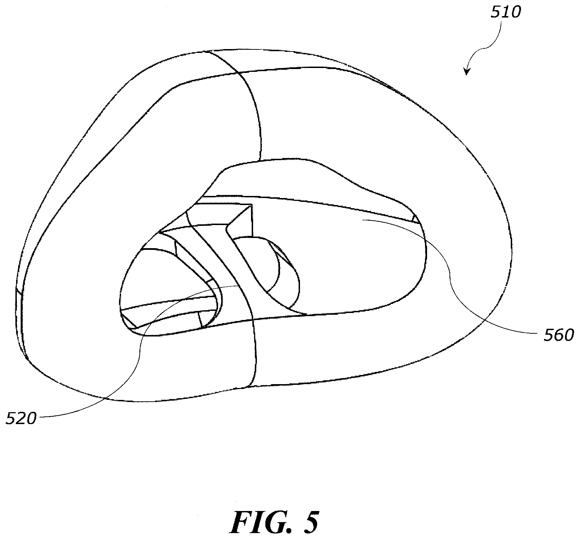

FIG. 5 illustrates a seal body 510 having an under-nose support 520. The seal body 510 is similar to the seal body 210 described in relation to FIGS. 1-4, and the seal body 510 may include all or some features of the seal body 210, and in some embodiments, the mask 200 may include the seal body 510 instead of the seal body 210. For example, the bridging portion 230 may be connected to the seal body 510. The under-nose support 520 of the nasal mask assembly can be sized, positioned and/or otherwise configured to contact at least a portion of an under-nose surface of the user's nose. The under-nose support 520 may extend within the mask cavity 560 that receives the user's nose in use and has a contact surface that is configured to contact at least a portion of the under-nose surface of the user's nose but without obstructing or completely obstructing the user's nostrils. Depending on the size of the user's nose, the under-nose support may contact a portion or portions of the under-nose surface without any obstruction of the user's nostrils or only partially obstructs one or both nostrils. The under-nose support 520 may include one or more extension or connection portions that extend from within the mask cavity 560 and connect to an edge of a contacting surface of the seal body 510 in an upper lip region of the seal body 510. The under-nose support 520 may vary in thickness across its length from a thinner end at or toward the end connecting to the face contacting portion of the seal body 510, to a thicker region at or near the opposing end. Various embodiments of the nasal mask interface and nasal seals of the interface with the under-nose support is further described in International Publication No. WO 2017/216708, published on December 21, 2017.

### Reinforcing Element

In some embodiments, a seal body of a mask, such as the seal body 210 or the seal body 510, may include a reinforcing element, which can provide additional support to the structure of the seal body and thus the nasal mask. The reinforcing element may reduce movement of the mask or seal body caused by the exertion of external force, thereby facilitating the maintenance of a suitable seal with the face of the user. For example, the reinforcing element may inhibit or at least partially prevent the movement of the seal body when the bridging portions 230 move, such as movement caused by movement of the facial pads 250. The movement of the bridging portions 230 are further described elsewhere herein. The reinforcing element may have any suitable shape or form, but the reinforcing element preferably provides sufficient stiffness such that it can prevent the seal body from folding, collapsing or buckling.

The reinforcing element may be positioned such that the reinforcing element does not contact the user when the mask is applied to the user. For example, the reinforcing element may be positioned at the anterior, non-patient facing side of the seal body, and/or within an interior space of the seal body. In some embodiments, the reinforcing element may be constructed from the same material as the seal body, but as a thickened portion. In some embodiments, the reinforcing element may be constructed from a different material than the seal body. In some embodiments, the reinforcing element may be formed integrally with the seal body (e.g. overmoulded). In some embodiments, the reinforcing element may be formed as a separate structure with the seal body and attached or otherwise installed to the seal body (e.g., removably or non-removably attached). The reinforcing element may be placed on the outer surface of the seal body, on the internal surface of the seal body, or within the seal body. The reinforcing element may be placed on an upper, lower, side portion, or any suitable location of the seal body.

FIGS. 6A-D illustrates a reinforcing element 226 placed within the seal body 210. As illustrated in FIGS. 6A-D, in one embodiment, the reinforcing element 226 has an elongate shape. In some embodiments, the reinforcing element 226 may extend at least partially across the width of the seal body 210. In some embodiments, the reinforcing element 226 may extend along the entire width or substantially the entire width of the seal body 210, from one side end to the other side end. In some embodiments, the reinforcing element extends from a portion of the seal body 210 where the seal body 210 connects with one of the bridging portions 230 to a portion of the seal body 210 where the seal body 210 connects with the other one of the bridging portions 230. As a result, the reinforcing element may maintain a general shape or width dimension of the seal body 210 or inhibit or prevent the seal body 210 from collapsing when the bridging portions 230 move toward the center of the seal body 210. In some embodiments, the reinforcing element 226 may be positioned along the lower portion of the seal body 210, such that the reinforcing element 226 does not interfere with rolling movement of the upper portion 212 described elsewhere herein or causes less interference than a position closer to or within an upper portion of the seal body 210.

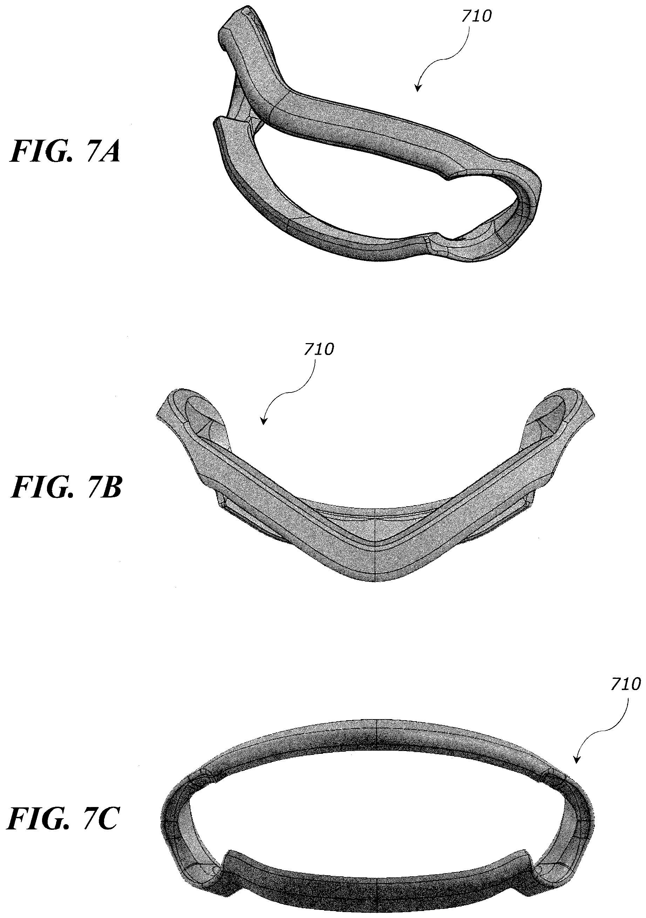

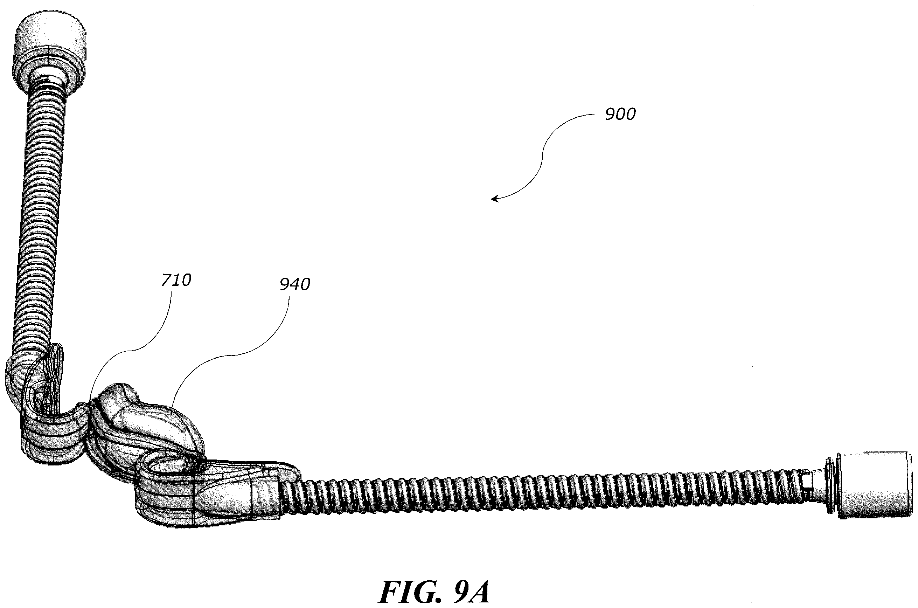

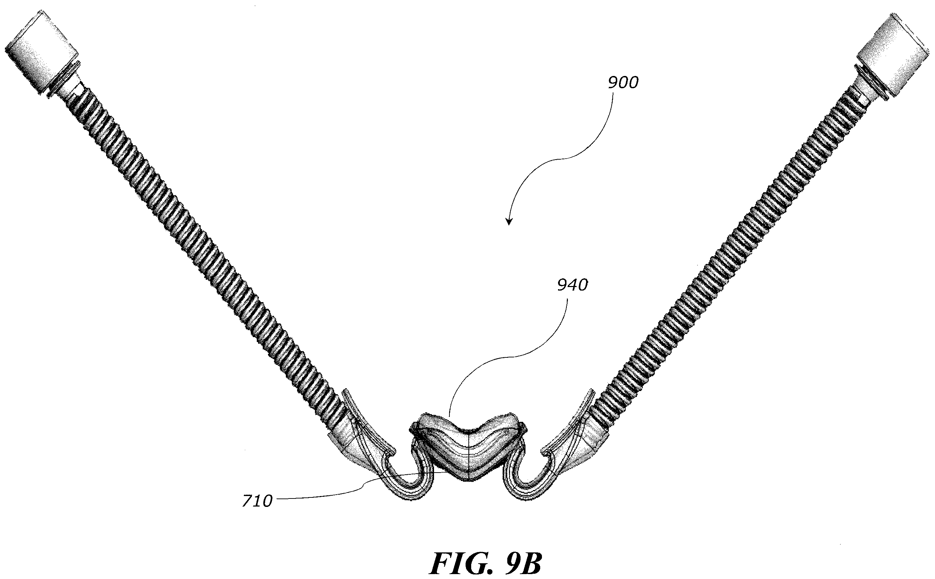

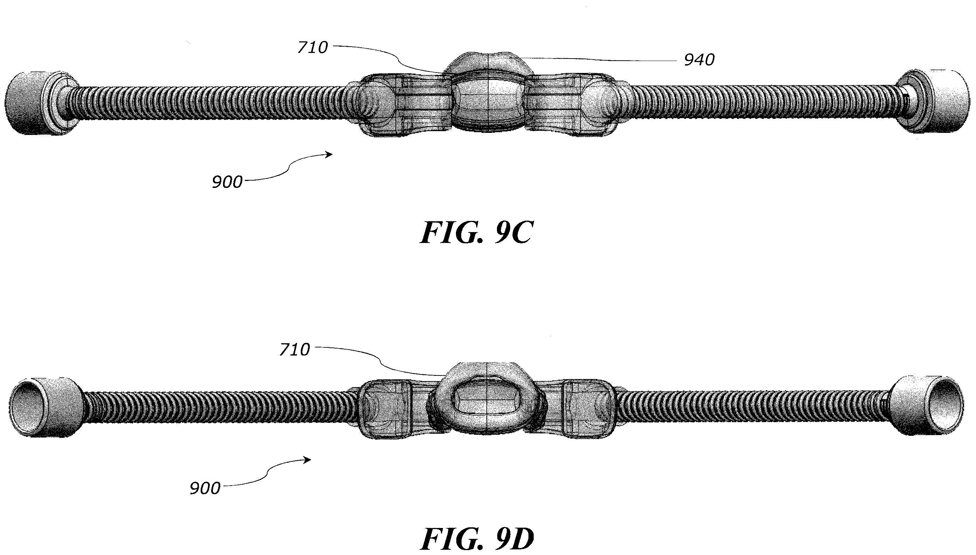

FIGS. 7A-7C illustrate a reinforcing clip 710, which is another example of the reinforcing element. In some embodiments, the reinforcing clip 710 has a substantially elliptical shape when viewed from the front. The reinforcing clip 710 is sized and shaped to fit externally over an anterior, non-patient side of the seal body. In some embodiments, the reinforcing clip 710 may be positioned internally within the mask cavity 260. FIGS. 8A-C illustrate a seal body 810 which can receive the reinforcing clip 710. In other respects, the seal body 810 can be the same as or similar to the seal bodies 210 and 510 described herein. The seal body 810 may include a receiving portion 820 designed to receive the reinforcing clip 710. For example, as illustrated in FIGS. 8A-C, an anterior, non-patient side of the seal body 810 is contoured to fit with the shape of the reinforcing clip 710. In some embodiments, the clip 710 can be removably coupled to the seal body 810, such that the clip 710 can be easily removed or replaced. In some embodiments, the reinforcing clip 710 may be attached to the seal body 810 by snap fitting or interference fitting. In some embodiments, the clip 710 may be fixedly attached to the seal body. In some embodiments, the clip 710 may be attached to the seal body by an adhesive. In some embodiments, the reinforcement clip 710 may be made of a stiffer material than the seal body. FIGS. 9A-D illustrate a patient interface 900 including the reinforcing clip 710. As shown in FIGS. 9A-9D, the reinforcing clip 710 may extend substantially around a front portion of a seal body 940.

FIGS. 8A-C further illustrate a single opening 815 which could be used to attach the one or more tubes, such as a coaxial tube, or two (or more) interface tubes (as described above) pre-assembled into a unitary connector for attachment to the opening.

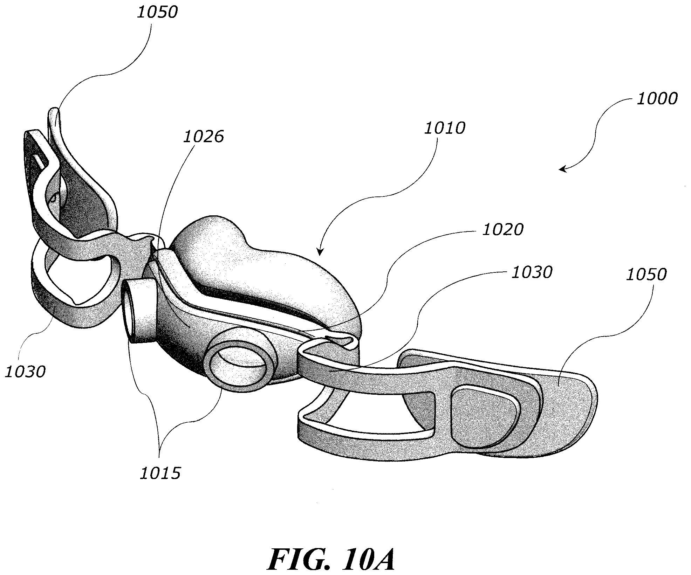

FIGS. 10A-B illustrate another mask 1000 including a seal body 1010. The mask 1000 can be substantially similar to the other masks described herein. Accordingly, features of the mask 1000 not described in detail can be the same as or similar to corresponding features of the other masks described herein, or can be of another suitable arrangement. Moreover, features of the mask 1000 can be utilized with the other masks described herein. The mask 1000 also includes a reinforcing clip 1020 as the reinforcing element. The reinforcing clip 1020 may be similar to the reinforcing clip 710, except that the reinforcing clip 1020 is integrally formed with the bridging portions 1030, such that the reinforcing clip 1020 extends towards or to the facial pads 1050. In some embodiments, the reinforcing clip 1020 and the bridging portions 1030 are made of the same material and can be an assembly or a unitary structure. In some embodiments, the reinforcing clip 1020 is thicker and the bridging portions 1030 are thinner, such that the reinforcing clip 1020 is stiffer than the bridging portions 1030. In other embodiments, the reinforcing clip 1020 can be made of a stiffer material than the bridging portions 1030.

In some embodiments, the seal body 1010 may not include a separate or distinct reinforcing element. In some such arrangements, certain regions of the seal body 1010 may be thickened, hardened or otherwise structured to reinforce the seal body 1010. For example, in some embodiments, a tube-receiving portion 1015 of the seal body 1010 and/or the end of the interface tubes is thickened or hardened and may function as the reinforcing element or in a manner similar to the reinforcing element. In some embodiments, the tube-receiving portion 1015 and/or a portion of the seal body 1010 adjacent to or surrounding the tube-receiving portion 1015 defines a reinforced region 1026 that is thicker, harder or otherwise reinforced relative to other regions of the seal body 1010, such that the reinforced region 1026 is stiffer than other regions of the seal body 1010. In some embodiments, the tube-receiving portion 1015 can be made of a stiffer material than other regions of the seal body 1010. In the illustrated arrangement, the reinforced region 1026 corresponds with a portion of the seal body 1010 bounded by the reinforcing clip 1020. With such an arrangement, the reinforced region 1026 of the seal body 1010 can assist the reinforcing clip 1020 in maintaining a desired shape of the seal body 1010. In other configurations, the reinforced region 1026 of the seal body 1010 can be configured to provide some or all of the desired reinforcement of the seal body 1010 such that the clip 1020 can be made more flexible, such as to reduce material costs and/or ease assembly.

### Securement System

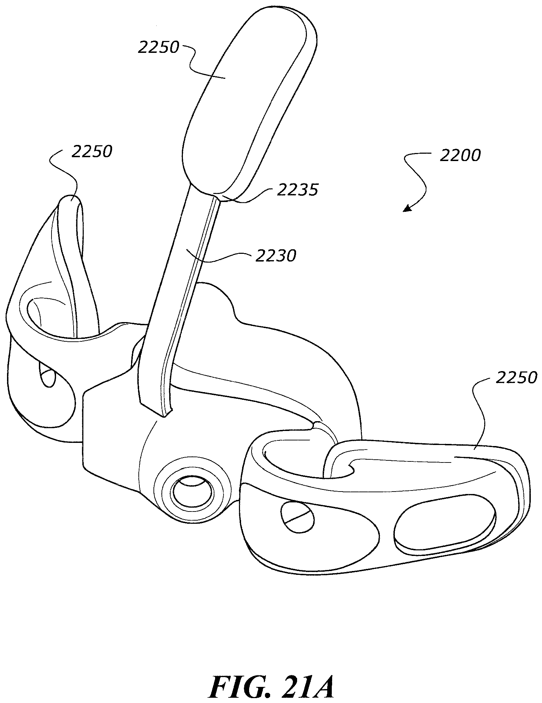

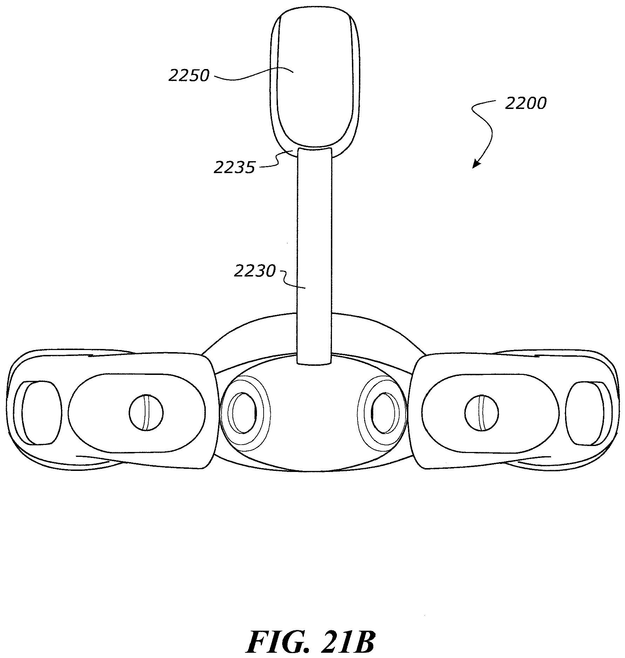

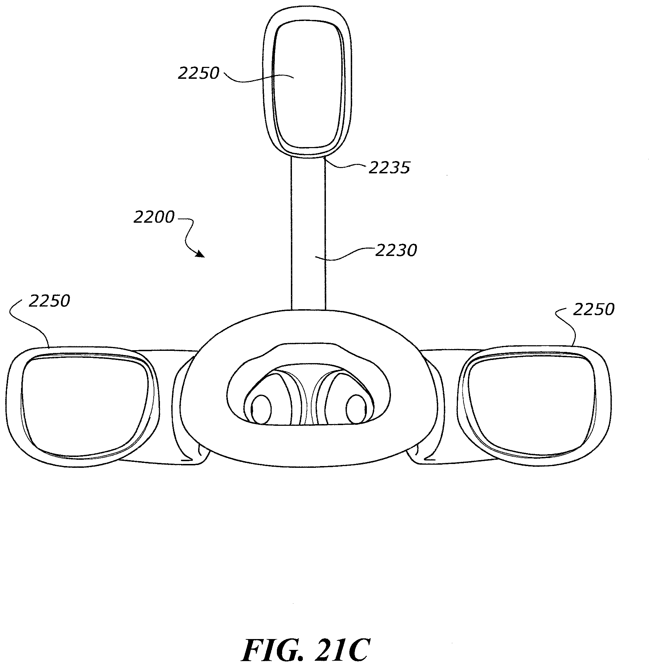

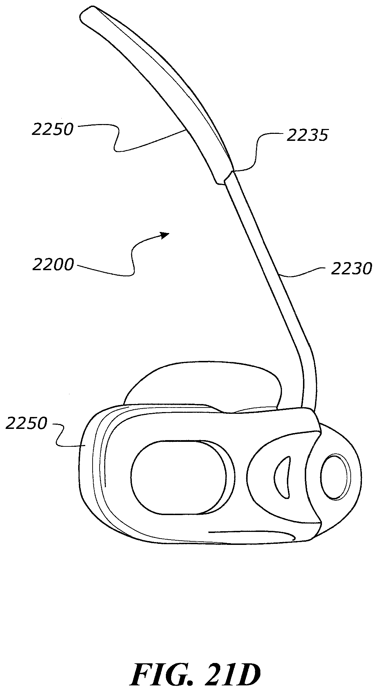

As illustrated in FIGS. 2A-D and 6A-D, the mask 200 includes a plurality of contoured backings or facial pads 250, which can rest on a user's face. The illustrated embodiment of the mask 200 includes a pair of the backing or facial pads 250, but in some embodiments, the mask 200 includes three or more backing or facial pads 250. For example, as shown in FIGS 21A-D, the mask 2200 may include a third backing or facial pad 2250 which can rest on the forehead area of or elsewhere on the user, in addition to the pair of backing or facial pads 2250 which rest on cheeks of the user's face. The backing or facial pads 250 may be connected to the end of the bridging portions 230. As illustrated in FIGS. 2A-D and 6A-D, each of the bridging portions 230 may terminate into the base or connecting portion 235 of the backing or facial pad 250. In some embodiments, the backing or facial pad 250 can receive and support one or more layers of the patch (e.g. a user interface patch or a dermal patch) or adhesives, such that the backing or facial pad 250 can rest on the user's face without directly contacting the user's skin when the user interface is secured on the user's face. The connection mechanism of the backing or facial pad 250 and the patches or adhesives is described in further detail elsewhere herein. In alternative arrangements, the backing or facial pad 250 can be connected to a headgear and/or a chinstrap for the securement of the mask 200 or the mask 200 can be otherwise connected to a headgear and/or a chinstrap, which are common components known in the art.

The backing or facial pads 250 may be pre-formed to be of a contour that is substantially curved to fit a user's face, cheek or upper lip region. In some embodiments with a point of contact in the forehead region, the contour could be selected to match or accommodate the contour of the user's forehead, which may be the same as, flatter, or more curved than a cheek region, for example. The enlarged end of the bridging portions 230 may be also contoured to accommodate the contour of the facial pads 250. The facial pads 250 may be anatomically shaped with a distribution and scale of curvature that reflects the facial geometry of the intended user. The anatomical shape of the facial pads 250 can give the interface a positive engagement with a user's face at a predetermined position where the contour of the facial pads 250 matches the user's facial contour. Pre-shaping or contouring the facial pads 250 to the user's facial features reduces the pressure applied to the user's face by any retention mechanism (adhesive tape, headgear or other means). This reduces the likelihood of pressure sores, head forming, or other injury. The positive engagement promoted by the anatomical shape of the facial pads 250 increases the stability of the mask 200 and therefore improves comfort and efficacy of the treatment being administered.

Figures 21A - 21D illustrate an embodiment of a mask 2200 with a third backing or facial pad 2250, configured to contact the patient's forehead region. As per the backing or facial pads 250 configured to rest on cheeks of the user, the backing or facial pad 2250 may be connected to the end of a bridging portion 2230. The bridging portion 2230 may terminate into a base or connecting portion 2235 of the backing or facial pad 2250. In some embodiments, the backing or facial pad 2250 can receive and support one or more layers of the interface patch or dermal patch, or adhesives, such that the backing or facial pad 2250 can rest on the forehead without directly contacting the skin. The connection mechanism of the backing or facial pad 2250 and the patches or adhesives can be the same as that of the backing or facial pads 250 arranged to lie on the cheeks, which is described in further detail elsewhere herein. The third backing or facial pad 2250 and bridging portion 2230 in conjunction with the associated interface patch/dermal patch/adhesives can provide vertical stability to the mask while positioned on the patients face and can assist in maintaining the mask seal in use. The third backing or facial pad 2250 and/or bridging portion 2230 can be fixed to or integrally formed with the mask 2200. Alternatively, the third backing or facial pad 2250 and/or bridging portion 2230 can be removably attached to the mask 2200. For example, the bridging portion 2230 may attach to the mask 2200 by a suitable fastener or may be removed by tearing or cutting the bridging portion 2230 from the mask 2200.

The patient interface may be secured to the patient's face using a securement system. The securement system may include a two-part releasable attachment or connection arrangement. The two-part releasable attachment or connection arrangement may be a two-part releasable attachment or connection arrangement as described in PCT application PCT/NZ2011/000218, published as WO2012/053910. The reference herein to a patient interface, patient interface patch, and similar terms, will be understood to be similar to the reference to a user interface or user interface patch in WO2012/053910. Such two-part releasable attachment or connection arrangement enables securement of the patient interface without use of a headgear or a bonnet, thereby preventing potential head forming by the headgear or the bonnet on babies or young children with still-developing skull structure and/or active fontanelles. The releasable connection arrangement includes a pair of patches that are affixed to the patient and the patient interface, respectively. The first patch is a dermal patch that is adhered or otherwise attached to the patient's skin. The dermal patch has a user side that faces the user's skin and an interface side that faces the patient interface. The user side of the dermal patch may be attached to the skin of a user by a dermatologically sensitive adhesive, such as a hydrocolloid. The patient interface side of the dermal patch is provided with the first part of the two-part releasable attachment or connection system.

The second patch is a patient interface patch. The patient interface patch also has a patient side and an interface side. The patient side of the patient interface patch is disposed adjacent the dermal patch when the system is engaged. The complimentary second part of the two-part releasable attachment or connection system is affixed to the patient side of the patient interface patch so that the respective parts of the two-part releasable attachment or connection system are easily engageable when the patches are brought together. The interface side of the patient interface patch is affixed to the patient interface (e.g. at the backing or facial pads 250). The patient interface patch may be integrated with, formed integrally with (i.e., by overmolding or other methods as are known in the art), or suitably adhered to the patient interface.

The two-part releasable attachment or connection arrangement may comprise a mechanical fastener, such as a hook and loop material (such as VELCRO^{™}), a magnet (or ferrous material to attract with a magnet) or an array of magnets disposed on the respective patches with the poles suitably arranged, an adhesive arrangement that is activated when the patches are urged together or another suitable releasable coupling. The interface side of the dermal patch may have one of a hook or a loop material, and the patient side of the patient interface patch may have the other of the hook or loop material, such that the dermal and patient interface patches are releasably attachable or connectable to each other.

Turning back to FIG. 2D, a rear surface 252 of the facial pad 250 can be initially provided without a patient interface patch, i.e., the surface 252 can receive or retain a patient interface patch. Such a patient interface patch may be connected to the rear surface 252 by an adhesive or other suitable connection as is known in the art (such as by hook-and-loop fastener, ultrasonic welding, and/or co-moulding or overmoulding). Once the patch is in position, it is ready to be connected to or receive a dermal patch. Alternatively, the patient interface patch may be assembled to the rear surface 252 of the facial pad 250 in the same operation as molding the facial pad 250. Embodiments with a third facial pad 2250 may be similarly provided with a two-part releasable attachment or connection arrangement such as that described above.

In some situations, a chinstrap may be used in conjunction with the two-part releasable attachment or connection arrangement. This chinstrap may be a standard or conventional chinstrap that is used to keep the patient's mouth closed during CPAP therapy. The chinstrap helps ensure a sealed system (by biasing the patient's mouth closed or substantially closed for eliminated, or at least reduced, mouth leak) and thus more effective treatment. In some embodiments, the chinstrap may be a part of a headgear or connected to the headgear which can be secured around the patient's head.

Additionally, or alternatively, a chinstrap may be part of the patient interface and may be used without a two-part releasable attachment or connection arrangement to secure the device.

In one example embodiment, the chinstrap comprises a material that is soft on the patient's face. Advantageously, the material may also be a stretchable fabric, such as a knit fabric, or a fabric comprising elastic material or elastic fibres. The chinstrap may have one or more regions of hook or loop material/fasteners, and/or may be substantially made up of or covered by hook or loop material. In one preferred embodiment, this is loop material, which is soft on the patient's face. In another example, this may be a hook material, so that any overlap of the user interface patch over the footprint of the strap does not cause any damage or scratching of the patient's face due to hooks on the interface patch. In an embodiment, the strap includes at least one region of hook material, and at least one region of loop material. The chinstrap may connect to a region of hook or loop fasteners on a connecting portion on the patient interface (e.g., the patient interface patch). This securement system secures the patient interface on the patient's face without having to apply any adhesive to the face. In one example embodiment, the chinstrap is used in combination with a continuous loop headgear that extends around the back and/or top of the patient's head. In a further example embodiment, the chinstrap comprises a slit or bifurcation to allow it to conform to the patient's curved chin region. In one embodiment, the chin strap may comprise, or may additionally comprise, an opening, slit, or bifurcation to hold a nasogastric or endotracheal tube.

The securement system may include a bonnet, such as an adjustable bonnet, which may include one or more sections of loop material. In some embodiments, the patient interface patch may be connected to the adjustable bonnet using a suitable structure, such as buttons, snaps, adhesive, magnets, ties, elastic, or other mechanical attachments. In some embodiments, the chinstrap may be attached to the bonnet via hook or loop fasteners. In some embodiments, the hook or loop fasteners on the bonnet or the chinstrap are movable or the bonnet or the chinstrap includes multiple hook or loop fasteners, such that the position where the patient interface or tube is secured on the bonnet or the chinstrap can be changed or adjusted as desired by the user or the clinician.

Furthermore, instead of extending over the chin, in one embodiment the at least one headgear strap may extend down the side of the patient's cheek. This type of strap may again comprise hook or loop material to attach to the patient interface and also to the bonnet (in an embodiment where the side strap portions and bonnet are not integral). In one preferred embodiment, this is loop material to be soft on the patient's face. In another example, this may be hook material, so that any overlap of the user interface patch over the footprint of the strap does not cause any damage or scratching of the patient's face due to hooks on the interface patch. In one embodiment, the strap comprises at least one region of hook material, and at least one region of loop material. An embodiment of these side strap portions may attach to the bonnet. The strap may have a longitudinal body that extends down the side of the patient's cheek.

Further examples of the securement system and its components are further described in PCT application PCT/NZ2011/000218, published as WO2012/053910.

### Structure and Movement of the Bridging Portions

As described herein, the facial pads (e.g. the facial pads 250) can rest directly or indirectly on a user's face when the mask (e.g. the mask 200) is worn by the user. Accordingly, the bridging portions (e.g. the bridging portion 230), which is connected to the facial pads, may be moved or deflected when the user's face moves. For example, when a portion of the user's face where the facial pads rest on moves, such external force or movement may be conducted to the bridging portion, and to the seal body (e.g. the seal body 210) eventually. In some instances, the bridging portions themselves may move or deflect when an external force is exerted on them. For example, the user of the mask may lay on the bridging portions, exerting force to the bridging portions, and thus to the seal body. Accordingly, it is desirable for the bridging portion to be constructed such that the seal body can maintain its seal with the user's face even when the user's face and/or the facial pads move.

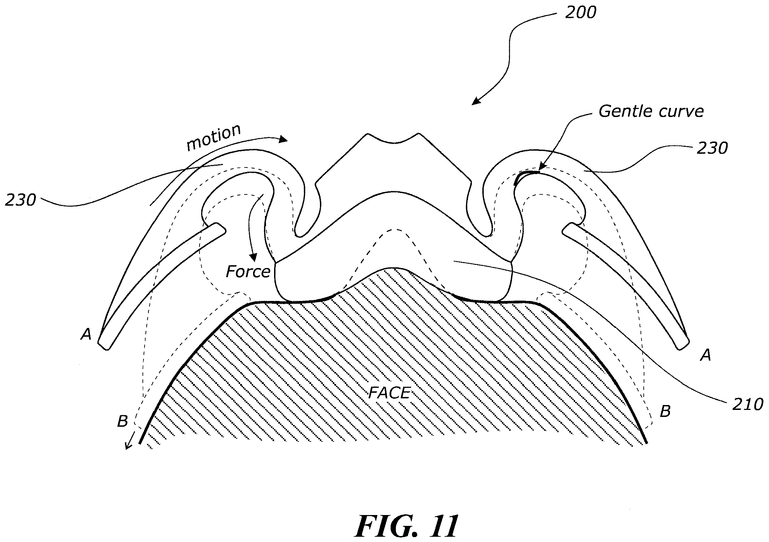

FIG. 11 illustrates the mask 200 positioned on a user's face. As illustrated, the bridging portions 230 may be constructed to have a first configuration (shown in solid line), which may be a relaxed or neutral configuration. In the first configuration of the bridging portions 230, the facial pads 250 are located in a first or neutral position (A). The bridging portions 230 are movable within a range of adjustment from the relaxed first configuration toward or to a second configuration (shown in dashed line), which may be a deformed or deflected configuration. In the second configuration of the bridging portions 230, the facial pads 250 are located in a second or adjusted or deflected position (B), which may be closer to the user's face than the first position (A). The second position (B) may place the facial pads 250 on the user's face (e.g., opposing cheek regions and/or an additional forehead region).

To apply the mask 200 to the user, the seal body 210 may be first positioned on the face of the user, such as on the user's nose as described herein with reference to FIG. 4, for example. With the bridging portions 230 in the first configuration and the facial pads 250 in the first position (A), the facial pads 250 may be spaced away from the user's face. With the seal body 210 positioned on the user's face, the facial pads 250 may be moved from the first position (A) toward the face of the user and toward or to the second position (B) until the facial pads 250 contact or rest upon the user's face. As described above, movement of the facial pads 250 from the first position (A) toward or to the second position (B) will likely cause movement (e.g., flexing or extension) of the bridging portions 230. The facial pads 250 can be secured to the user's face directly or indirectly by an external force, such as using an adhesive connection, a headgear arrangement, a two-part connection arrangement as discussed above, or another suitable support structure, such as any of those described herein.

When the facial pads 250 and the bridging portions 230 are in position (B) illustrated in FIG. 11, the bridging portions 230 preferably generate a force tending to apply downward pressure (in the orientation of Figure 11) to the seal body 210, which helps create or maintain a seal between the seal body 210 and the user. The force is generated as a result of deformation of the bridging portions 230 from their first or relaxed configurations and the resulting material strain tending to revert the bridging portions 230 back towards their first or relaxed configurations.

Advantageously, if the first configuration of the bridging portion 230 spaces the respective facial pad 250 away from the user's face in position (A) as illustrated in FIG. 11, the user or a clinician is provided with the freedom to fit and position the facial pads 250 on the user's face as desired. Such an arrangement facilitates adjustment or customization of the fit of the mask 200. For example, extending or deforming the bridging portions 230 in an outward direction before securing the facial pads 250 increases the downward force acting on the seal body 210 in comparison to simply move the facial pads 250 straight towards the user's face. The bridging portions 230 may be constructed to limit downward force to an acceptable level to inhibit or prevent the user or the clinician from overtightening the mask 200 by way of overextension of the bridging portions 230, while retaining some adjustability to reduce, eliminate or otherwise address leaks that may occur.

In some configurations, the deformable configuration of the bridging portions 230 provides some degree of de-coupling of the facial pads 250 from the seal body 210, preferably while maintaining the downward force on the seal body 210 toward the face of the user. For example, deformation of the bridging portions 230 may allow cheek movement of the user to have a reduced or minimal effect on the downward pressure between the seal body 210 and the bridging portions 230 in comparison to a straight or substantially straight bridging portion. Preferably, the deformation of the bridging portions 230 at least reduces or possibly eliminates disruption of the seal between the seal body 210 and the user's face as a result of cheek movements of the user's face.

Some or all of these advantages may be provided by the shape of the bridging portions 230, the orientation of the bridging portions 230 or the material(s) used to construct the bridging portions 230. In some embodiments, the bridging portions 230 may be made of a flexible or elastically-deformable material, such that at least a portion of a length of the bridging portion 230 has flexibility and/or elasticity. In some embodiments, the bridging portions 230 are each shaped such that its curved geometry facilitates the flexibility of the bridging portions 230. The flexibility of the bridging portions 230 preferably allows the bridging portions 230 to absorb forces exerted on them, for example caused by the movement of the user's cheek. Accordingly, as described above, only some of the force exerted on the facial pads 250 and, thus, the bridging portions 230 is transferred to the seal body 210. Advantageously, such an arrangement assists in maintaining the seal between the seal body 210 and the user's face during movement of the user's cheeks.

As illustrated in, for example, FIG. 2B and FIG. 11, the bridging portions 230 may be provided as elongate arms shaped to have or define a non-linear, circuitous or serpentine path 300 between a first location or first end 302 at a junction between the seal body 210 and the bridging portion 230 and a second location or second end 304 at a junction between the bridging portion 230 and the base 235 of the associated facial pad 250. In some configurations, the bridging portions 230 each define an S-shape as viewed from above. The path 300 can be defined by a geometric center of the bridging portion 230 along its length. Following the path 300 from the first end 302 to the second end 304, the path 300 preferably defines at least a forwardly-extending portion followed by at least an outwardly or rearwardly-extending portion relative to the overall mask 200 wherein a forward direction is toward the front of the mask or away from the user and the rearward direction is toward the rear of the mask or toward the user. In addition, although discussed in terms of the path 300, it will be understood that the references to the orientation of the path 300 equally apply to the orientation of the bridging portion 230 itself. Thus, any reference to the path 300 can be replaced with a reference to the bridging portion 230.

In the illustrated arrangement, the path 300 has an initial outward trajectory when moving along the path 300 in a direction from the first end 302 toward the second end 304. The path 300 then extends in a substantially forward direction. In some configurations, the initial outward trajectory can be omitted and the path 300 can initially extend in a substantially forward direction from the first end 302. In the illustrated arrangement, the path 300 continues in a forward direction while moving inwardly toward a centerline 306 of the mask 200. The path 300 then moves outwardly away from the centerline 306 while still moving in a forward direction. The illustrated path 300 then reverses course and moves rearwardly. In addition, the path 300 also moves outwardly away from the centerline 306 while moving rearwardly to the second end 304. Overall, the illustrated path 300 extends greater in a forward direction than in a rearward direction, thereby the second end 304 of the illustrated path 300 is positioned at least partially forwardly than the first end 302. Accordingly, an imaginary straight line extending from the first end 304 to the second end 302 forms an angle 308 smaller than 90°. In some embodiments, the angle 308 can be between about 30°-80° inclusive, and including any and all values within this range (such as about 30°, 35°, 40°, 45°, 50°, 55°, 60°, 65°, 70°, 75° or 80°). However, the angle 308 can be changed when the bridging portions 230 are extended or deformed. For example, at the second or adjusted or deflected position (B) of FIG. 11, the angle 308 can be equal or greater than 90°.

Thus, the illustrated path 300 changes direction at least once in a fore-aft direction or a direction along the centerline 306 (e.g., moves forwardly and then moves rearwardly). Preferably, the path 300 also changes direction at least once in a direction toward and away from the centerline 306. In particular, the illustrated path 300 changes direction twice in the direction toward and away from the centerline 306 (e.g., moves away from, then toward and then away from the centerline 306).

Such an arrangement increases the length of the path 300 or the effective length of the bridging portion 230, in a space-efficient manner, thereby lessening the bulkiness of the mask 200 and improving the comfort of the user. A greater effective length of the bridging portions 230 can provide the bridging portions 230 a greater range of movement or adjustment, such that the mask 200 can be applied to users with a wider range of face sizes. In addition or in the alternative, a greater effective length provides the bridging portions 230 with a flatter force-extension curve, which may inhibit or prevent the seal body 210 from exerting too much force on the user's face even when the bridging portions 230 are significantly stretched while in use. A flatter force-extension curve provides a more constant sealing force on a patient's face during cheek movement.

In some configurations, the effective length of the bridging portion 230 or length of the path 300 is significantly greater than one or both of a linear distance 310 between the first end 302 and the second end 304 of the bridging portion 230 and a lateral distance 312 between the first end 302 and the second end 304 of the bridging portion 230 (in a direction perpendicular to the centerline 306). In general, the larger the ratio between the effective length or length of the path 300 and the linear distance 310 or lateral distance 312, the larger the adjustment range, the flatter the force-extension curve and/or the greater the de-coupling of movement between the facial pads 250 and the seal body 210. In some embodiments, each of the bridging portions 230 may have a length or arclength (length of the path 300) of between about 150%-300%, inclusive, and including any and all values within this range (such as about 170%, 175%, 200%, 210%, 225%, 250% or 275%), of either of the linear distance 310 or the lateral distance 312. In some embodiments, ratio of the length of the path 300 to either of the linear distance 310 or the lateral distance 312 can be greater.

As shown in FIGS. 2B, 6B and 11, the bridging portions 230 may have a relatively gentle curve/radius. In some such configurations, the bridging portions 230 roll in and out, in contrast to folding like a hinge, during movement. In some embodiments, the bridging portions 230 have a radius of curvature 320 of between about 0.5cm-30cm, inclusive, including any value within this range, such as about 0.75cm, 1cm, 2cm, 5cm, 10cm, 15cm, 20cm, 25cm or 30 cm, at any location along the length of the bridging portion 230. In some embodiments, the radius of curvature 320 can be greater. In some embodiments, each of the bridging portions 230 may have substantially constant or smoothly varying curvature throughout its length, such that the bridging portions 230 do not include an abruptly bent or folded point. In some embodiments, each of the bridging portions 230 may have at least partially an elliptical shape, a circular shape, a hook shape, a U-shape, or an S-shape as viewed from above or below the mask 200. In some embodiments, as shown in FIGS, 2B, 6B and 11, each of the bridging portion 230 may have a portion curved inward relative to the seal body 210, and portion curved outward relative to the seal body 210. In some embodiments, the bridging portions 230 may have an at least partially circular or elliptical cross-section.

In some embodiments, the bridging portions 230 may be made of a flexible material, such that the bridging portions 230 are flexible or deformable, as described above. In some embodiments, the bridging portions 230 may be made of the same material as the seal body 210 in an assembled or unitary construction. In some embodiments, the bridging portions 230 may be made of one or more elastomer materials, such as silicone, rubber, polyethylene, etc. In some embodiments, the bridging portions may be made of a single material. In some embodiments, the bridging portions 230 may be made of two or more materials, such that a region of the bridging portion 230 has a different flexibility relative to another region.

In some embodiments, each of the bridging portions 230 may be shaped such that its geometry contributes to the flexibility. In some embodiments, the bridging portions 230 may have a constant or substantially constant cross-sectional area throughout their length. In some embodiments, the bridging portions 230 may have a varying cross-sectional area along their length, such that, for example a region of the bridging portion 230 has greater cross-sectional area and, assuming the same material, is stiffer than another region.

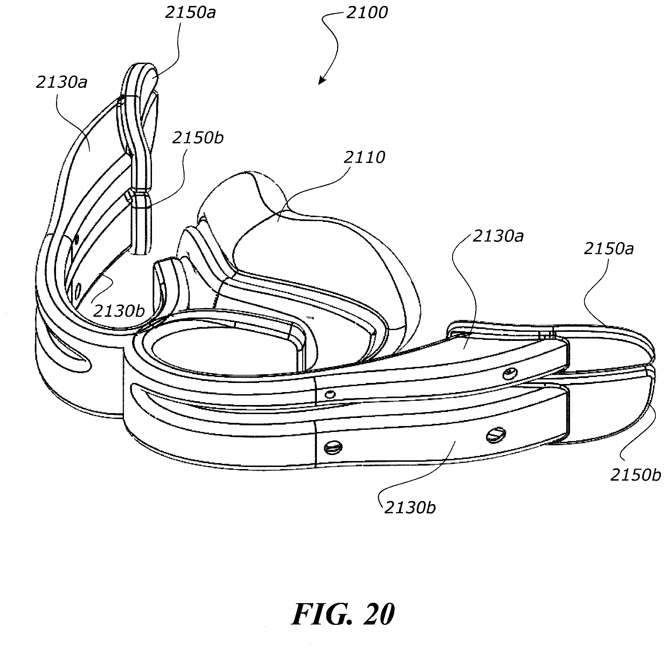

In some embodiments, as illustrated in FIG. 11, each of the bridging portions 230 is attached to the seal body 210 separately, thereby allowing movement independent from each other. For example, when one cheek and thus one facial pad 250 and one bridging portion 230 are moved (e.g. when the user is lying on his side or when the user's face is squeezed), the other bridging portion 230 may not move, and a sufficient seal may be maintained. In other embodiments, such as shown and described with reference to FIG. 20, for example, the bridging portions 230 may be connected to one another, which may provide additional rigidity where desired in comparison to fully independent bridging portions 230.

Other configurations of bridging portions are also possible, many of which share some or all of the characteristics and/or provide similar functionality as described above. For example, the mask 1000 illustrated in FIGS. 10A-B includes bridging portions 1030 connected to the reinforcing clip 1020. The mask 1000 further includes facial pads 1050 attached to the bridging portions 1030. The facial pads 1050 may be similar to the facial pads described elsewhere herein, such as the facial pads 250. The bridging portions 1030 can have an overall curvature in a relaxed position that creates a substantially longer effective length relative to the linear distance or lateral distance. In the illustrated arrangement, each of the bridging portions 1030 changes direction at least twice in a fore-aft direction. For example, the bridging portions 1030 each extend rearwardly, forwardly and then rearwardly moving from an end adjacent the seal body 1010 to an end adjacent the facial pad 1050. In the illustrated arrangement, the bridging portions 1030 move continuously outwardly from the end adjacent the seal body 1010 to the end adjacent the facial pad 1050; however, changes in direction could also occur in this direction.

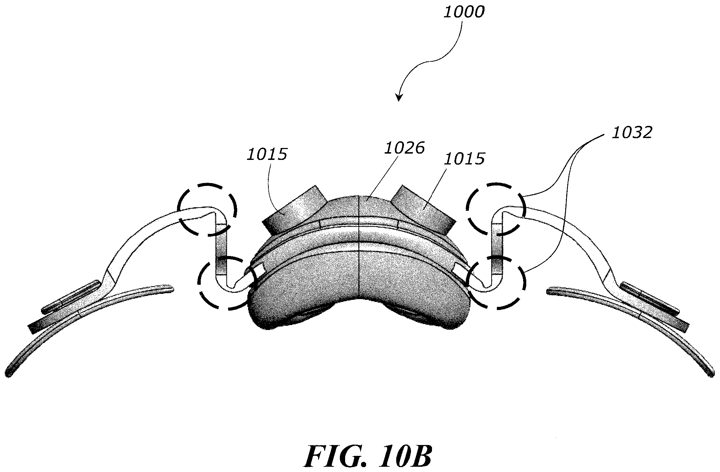

As described with regard to the mask 200, forces may be exerted to the facial pad 1050 and the bridging portions 1030, for instance, when the patient is lying on the side of his or her face or when the patient's face is squeezed. When such forces are exerted on the facial pads 1050, the bridging portions 1030 are inclined to fold inward at the hinges 1032, labeled in FIG. 10B. Such inward bending of the bridging portions 1030 at the hinges 1032 may press the seal body 1010 toward the user. The bending of the bridging portion hinge 1032 can be limited by the patient's anatomy. A variety of hinge types can be used for the hinges 1032. The hinges can bend in a predictable, limited number of directions to define the mechanical behavior of the dynamic interface.

In some embodiments, a strap may be used in place of the bridging portions. The strap may be integrally formed with the seal body, or may be a separate piece and can be attached to the seal body. In some embodiments, the strap may have regions of hook or loop material for securing the mask to the securement system described elsewhere herein. In some embodiments, the strap can be made of, or covered in, fabric or a fabric/foam laminate such as neoprene or breath-o-prene.

### Tube Attachment and Positioning

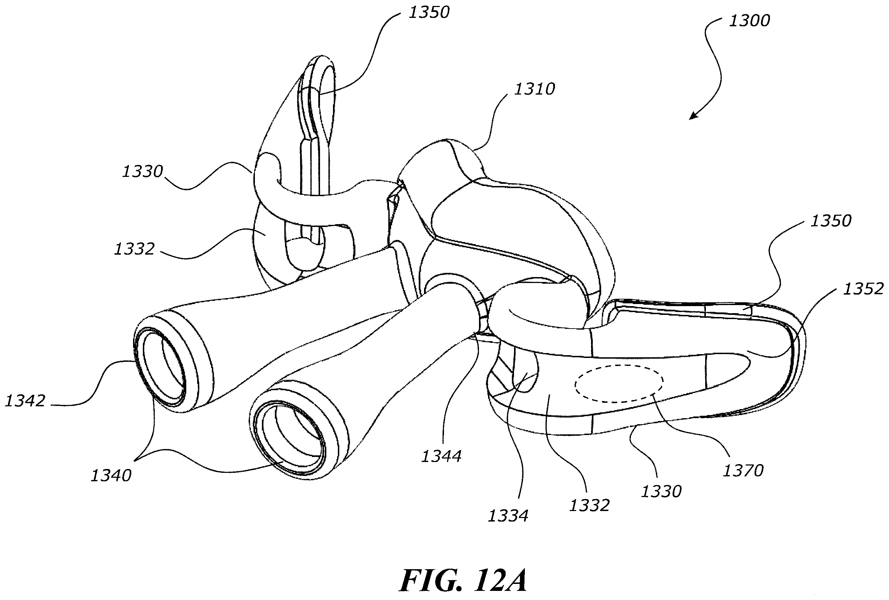

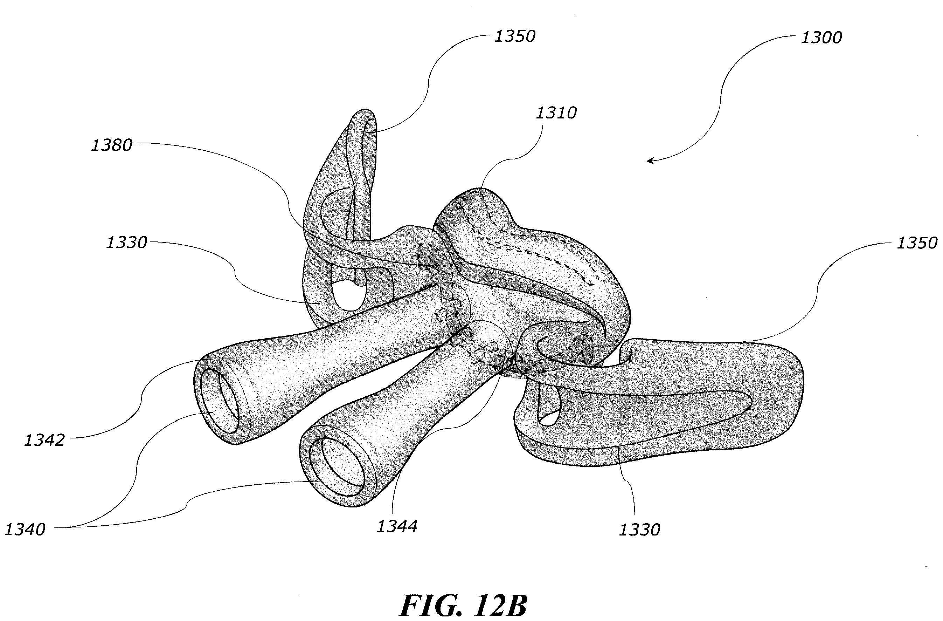

FIGS. 12A-E illustrate a mask 1300 including a seal body 1310, bridging portions 1330, and facial pads 1350. As shown in FIGS. 12B-E, the mask 1300 may also include a reinforcing element 1380 similar to the reinforcing element 226. The mask 1300 and its components can be the same as or similar to the masks and their corresponding components disclosed elsewhere herein, such as the mask 200 or 1000, except as described below. In some embodiments, the seal body 1310 includes two tube extensions 1340 as shown in FIGS. 12A-E. Each of the tube extensions 1340 extends from a seal end 1344 adjacent the seal body 1310 toward a tube end 1342 away from the seal body 1310 in a direction away from the user's face when the mask 1300 is worn. The tube extensions 1340 can connect interface tubes to the seal body 1310, allowing gas flow from and to the user.

In some embodiments, the tube extensions 1340 may be integrally formed with the seal body 1310. In some embodiments, the tube extensions 1340 may be connected to the seal body 1310. The seal body 1310 may include openings at the front portion, such that the tube extensions 1340 can be connected at the openings. In some embodiments, each of the seal ends 1344 may have elliptical or circular shape. In some embodiments, the tube extensions 1340 may be removably attached to the seal body 1310, such that the tube extensions 1340 can be attached or removed as desired. When the tube extensions 1340 are removed, the seal body 1310 may have a tube-receiving portion such as the tube-receiving portion 214, such that the seal body 1310 can receive interface tubes. In some embodiments, the seal end 1344 can be connected to the seal body 1310 at the tube-receiving portion. In some embodiments, the seal end 1344 can be connected to the seal body 1310 by suitable connection mechanisms, such as friction-fitting, snap-fitting, screw-fitting or barbed connections. The connection between the seal end 1344 and the seal body 1310 preferably is sealed such that the gas passing to or from the seal body 1310 does not leak.

The tube end 1342 of each of the tube extensions 1344 can be connected to any suitable interface tubes. In some embodiments, the interface tubes are breathable, being permeable to water vapor while being impermeable to liquid or bulk flow of gases. Further examples of interface tubes which can be used with the patient interfaces described herein are described in U.S. Patent Application No. 15/514,221. In some embodiments, an adaptor or connecter may be placed between the tube end 1342 and the interface tube, such that various interface tubes can be connected to the tube extensions 1340. The tube extensions 1340 may be removably attached to the interface tubes by any suitable connection mechanisms, for example friction-fitting, snap-fitting, interference-fitting, screw-fitting or barbed connections.

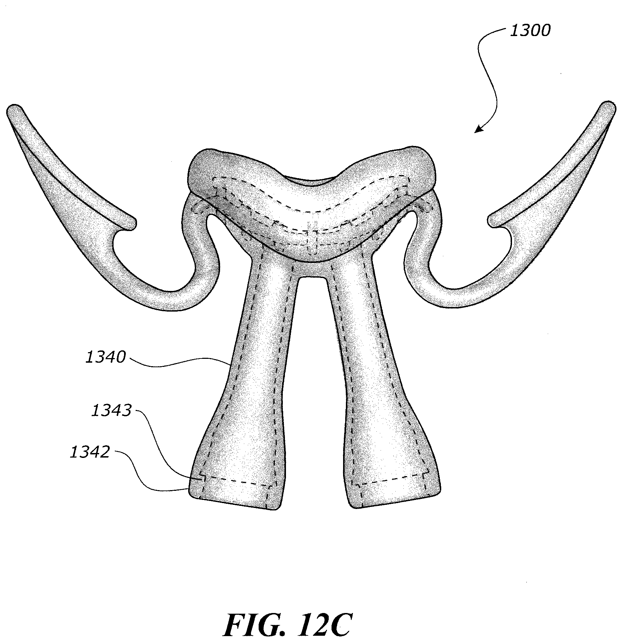

FIG. 12C illustrates internal pathways of the tube extensions 1340 according to some embodiments. As shown in FIG. 12C, the tube end 1342 of each of the tube extensions may include an inner flange 1343. The inner flange 1343 can receive and hold the interface tubes in place. The connection between the tube end 1344 and the interface tube may be sealed such that the gas passing to or from the mask 1300 does not leak.

In some embodiments, each of the tube extensions 1340 taper towards the seal end 1344 and have a smaller diameter at a region adjacent the seal end 1344 than a region adjacent the tube end 1342. In some embodiments, each of the tube extensions 1340 may taper away from the seal body 1310 and have a greater diameter at a region adjacent to the seal end 1344 than a region adjacent to the tube end 1342. The tapered shape of the tube extensions 1310 may allow connection of larger interface tubes than what could be or is desirable for accommodated directly on the seal body 1310. For example, interface tubes having a large diameter can be connected to a smaller mask via tube extensions 1340. In other arrangements, the tube extensions 1340 may have a constant or substantially constant diameter throughout their length. In some embodiments, the seal end 1344 and the tube end 1342 of the tube extensions 1340 can have the same or substantially the same size and shape.

The tube extensions 1340 may be flexible such that the tube extensions can be moved or positioned or bent (without occluding) towards locating features which may be provided in the form of grooves 1332. The flexibility of the tube extensions 1340 can facilitate comfort of the user and thereby facilitates more sleeping positions, and kangaroo care positions. At the same time, the tube extensions 1340 may have some structural integrity, such that the tube extensions 1340 do not collapse or kink in use. In some embodiments, the tube extensions 1340 may have ribs or supports internally and/or externally to prevent collapse while maintaining flexibility.

In some embodiments, to reduce the bulkiness of the patient interface, the tube extensions 1340 can be bent and placed at least partially along the bridging portions 1330. In some embodiments, each of the bridging portions 1330 includes a locating feature, which in some embodiments is a groove 1332 extending at least partially along its front surface. The groove 1332 is configured to receive a portion of the tube extensions 1340. In some embodiments, the bridging portions 1330 may further include cutouts 1334 at the groove 1332. The tube extensions 1340 may be long enough to fit into the grooves 1332 and the cutouts 1334. In some embodiments, the tube extensions 1340 are shorter, and interface tubes connected to the tube extensions 1340 are received in the groove 1332. In some embodiments, the tube extensions 1340 or the interface tubes may further extend over a front, non-face side of the facial pads 1352. In some embodiments, the front, non-face side 1352 of the facial pads 1350 include hook or loops which can receive hook or loops attached to the tube extensions 1340 or the interface tubes to retain them in a desired position. In some embodiments, the facial pads 1350 may include hook or loop material or adhesives on both sides (patient-facing and away-from-patient facing), such that the facial pads 1350 can be attached to the tube extensions 1340 or the interface tubes at one side, and the securement system described elsewhere herein on the other side.

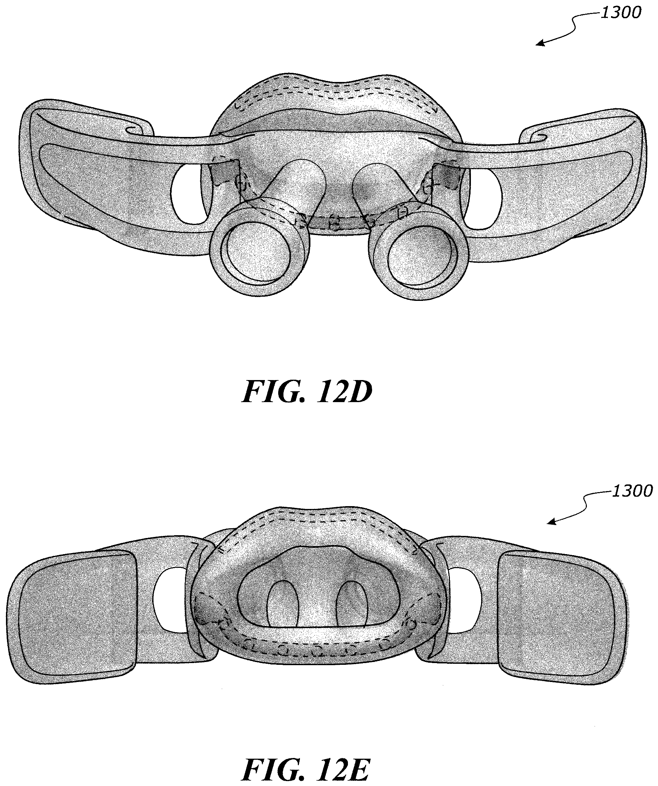

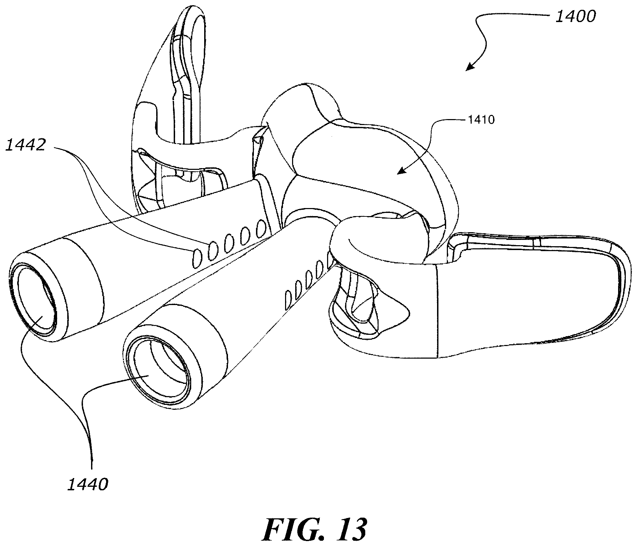

FIG. 13 illustrates a mask 1400 similar to the mask 1300 described with regard to FIG. 12 except that a tube extension 1440 includes one or more recessed portions 1442 with reduced wall thickness and stiffness for facilitating bending the tube extension 1440 without kinking. In the illustrated arrangement, the plurality of recessed portions 1442 are aligned with one another in a lengthwise direction of the tube extensions 1440, but the recessed portions may be arranged to facilitate bending of the tube extensions 1440 in any desired directions. The tube extensions such as the tube extensions 1340 or 1440 may include any additional structure other than recessed portion, for facilitating bending without kinking or folding.

In some embodiments, the tube extensions 1440 can each include openings for venting of exhaust gases from the mask 1400. For example, the openings can take the place of the recessed portions 1442. The openings 1442 can form a bias flow vent, as is known in the art. In some configurations, the openings 1442 can also facilitate bending of the tube extensions 1440 in a manner similar to the recessed portions 1442 described above. In addition or in the alternative, a suitable vent arrangement can be provided on another portion of the mask 1400, such as the seal body 1410.

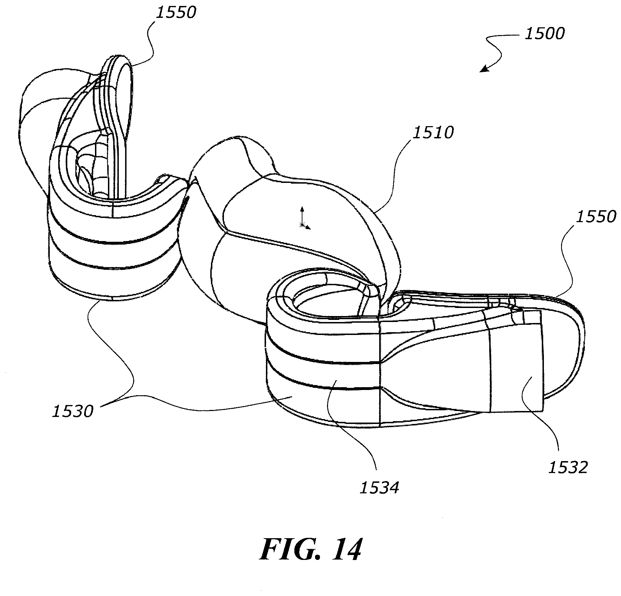

In some embodiments, the interface tubes may be connected to bridging portions, instead of being directly connected to the seal body or via the tube extensions. FIG. 14 illustrates a mask 1500 having a seal body 1510, bridging portions 1530, and facial pads 1550. The mask 1500 is similar to the masks described herein, such as the mask 200 or the mask 1300, except as described below.

In some embodiments, each of the bridging portions 1530 includes or defines a substantially U-shaped portion as viewed from above. Preferably, the bridging portions 1530 are configured to provide similar functionality and one or more of the advantages of the other bridging portions described herein. For example, the bridging portions 1530 can generate a sealing force on the seal body 1510 in use. The bridging portions 1530 can provide at least some de-coupling of the facial pads 1550 from the seal body 1510. Each of the bridging portions 1530 can define a path length or an effective length that is greater than a (e.g., lateral or linear) distance between the seal body 1510 and the associated facial pad 1550. Such an arrangement can provide the bridging portion 1530 with a relatively flat force-extension curve, as described above.

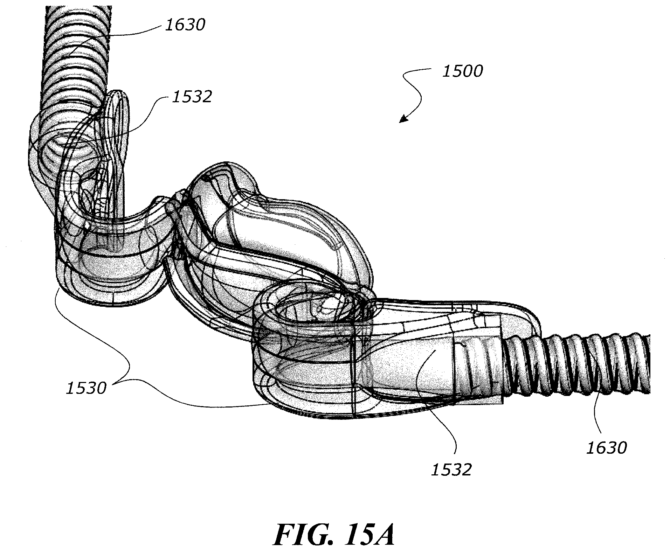

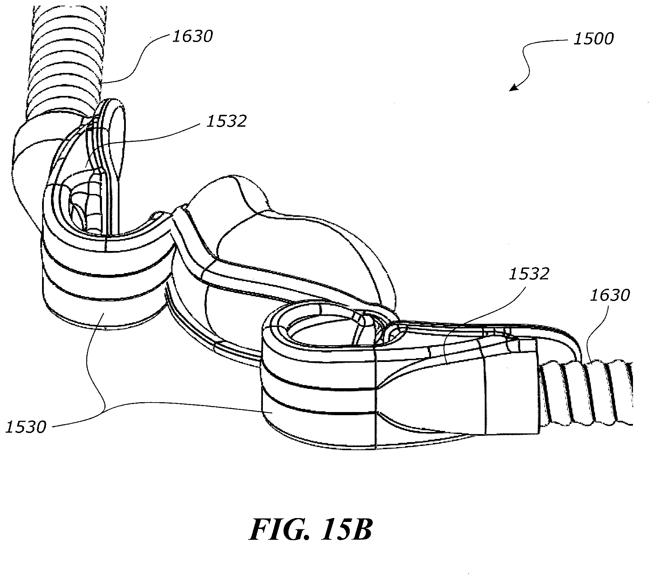

In some embodiments, each of the bridging portions 1530 includes a tube receiving opening 1532 as illustrated in FIG. 14. Each of the bridging portions 1530 may further have an internal gas pathway 1534 connecting the tube receiving opening 1532 and a mask cavity of the seal body 1510. The internal gas pathway 1534 may be connected to an internal opening (not shown) at the seal body 1510 such that the internal gas pathway 1534 fluidically connects the tube receiving opening 1542 to the mask cavity of the seal body 1510 while being internally located within the assembly of the bridging portions 1530 and the seal body 1510 along a portion or an entirety of its length. The tube receiving opening 1532 may be constructed such that the interface tube is connected to the bridging portion 1530 close to the user's face. FIGS. 15A-B illustrates the mask 1500 with the interface tubes 1630 attached to the bridging portions 1530. By having an internal gas pathway and the interface tube 1630 connected closely to the user's face, the mask 1500 may be less bulky and provide increased comfort to the user. The interface tube 1630 may be connected to the tube receiving opening 1532 by any suitable mechanism, such as any mechanism described herein. For example, the tube receiving opening 1532 may have an inner flange and can receive a barbed insert of the tube 1630. In some configurations, the barbed insert of the tube 1630 can be connected to the tube 1630 by overmoulding.

In some embodiments, the bridging portions 1530 may have overall contoured shapes as described with respect to the bridging portions 230, but to accommodate the interface tube at the tube receiving opening 1532, the bridging portions 1530 may have greater height along their lengths than the bridging portions 230. In some embodiments, the bridging portions 1530 have a height substantially similar to the diameter of the tube receiving openings 1532. In some embodiments, the bridging portions 1530 have a height greater than the diameter of the tube receiving opening 1532. The bridging portions 1530 may have a substantially similar or smaller height than the seal body 1510. At the same time, the bridging portions 1530 may have similar thickness with the bridging portions 230, such that the bridging portions 1530 have laterally rolling movement of the bridging portions described elsewhere herein. Accordingly, the bridging portions 1530 may have a flatter cross-section than the bridging portions 230.

The internal gas pathway 1534 may have small width/diameter, such that it does not interfere with the movement of the bridging portion 1530. For example, the internal gas pathway 1534 may have a diameter of about 2mm-1 cm or greater. As illustrated in FIG. 14, the tube receiving opening 1532 may include a raised portion to accommodate the diameter of the interface tube. At the same time, the bridging portions 1530 may have a certain degree of some structural integrity, such that they do not collapse or kink in use. In some embodiments, the bridging portions 1530 may have ribs or supports internally and/or externally to prevent collapse while maintaining flexibility.

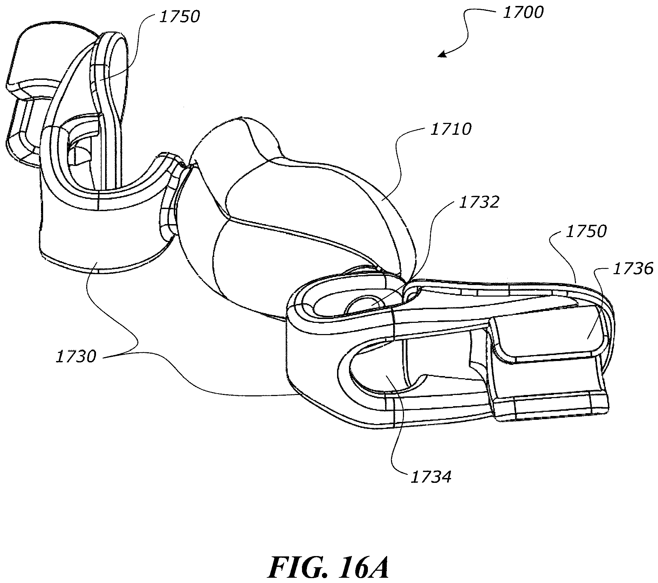

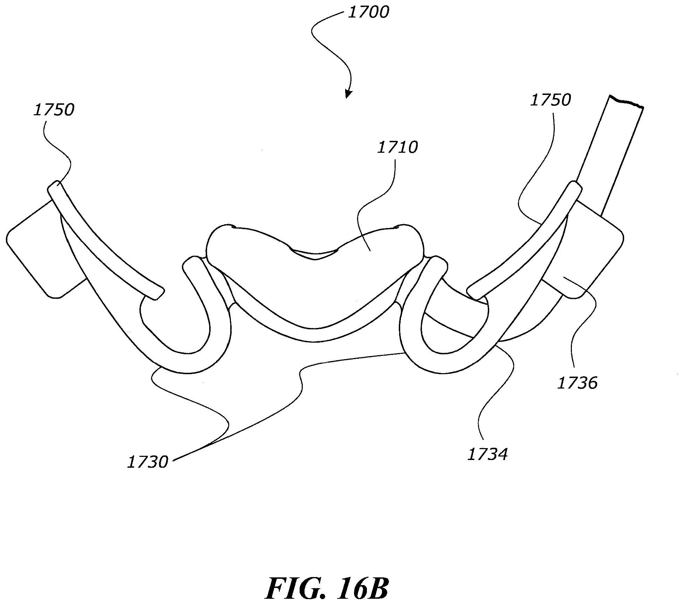

FIG. 16A illustrates a mask 1700 similar to the mask 1500 described in relation to FIGS. 14-15B. The mask 1700 includes a seal body 1710, bridging portions 1730, and facial pads 1750. In some embodiments, at least one of the bridging portions 1730 include a tube receiving opening 1732. The bridging portions 1730 may be similar to the bridging portions 1530, but the tube receiving opening 1732 may be defined in the bridging portion 1730 adjacent at a location where the bridging portion 1730 is connected to the seal body 1710. The bridging portions 1730 may each further include one or more cutouts 1734 such that an interface tube may be passed through the cutout 1734 and connected to the tube receiving opening 1732 as illustrated in FIG. 16B. The bridging portions 1730 may each include a clip 1736 which can removably retain the interface tube, such that the interface tube can be kept close to the user's face. The clips 1736 may isolate the mask seal from any forces that are exerted on the opposing end of the tubes. In some embodiments, the clip 1736 may be positioned at the facial pad 1750 or at the bridging portion 1730 adjacent the facial pad 1750. In some embodiments, the bridging portion 1730 may include two or more clips 1736. In some embodiments, the bridging portion 1730 may include tube retaining mechanism in addition to or alternative to the clip 1736, for example, adhesive or a hook- and-loop fastener.

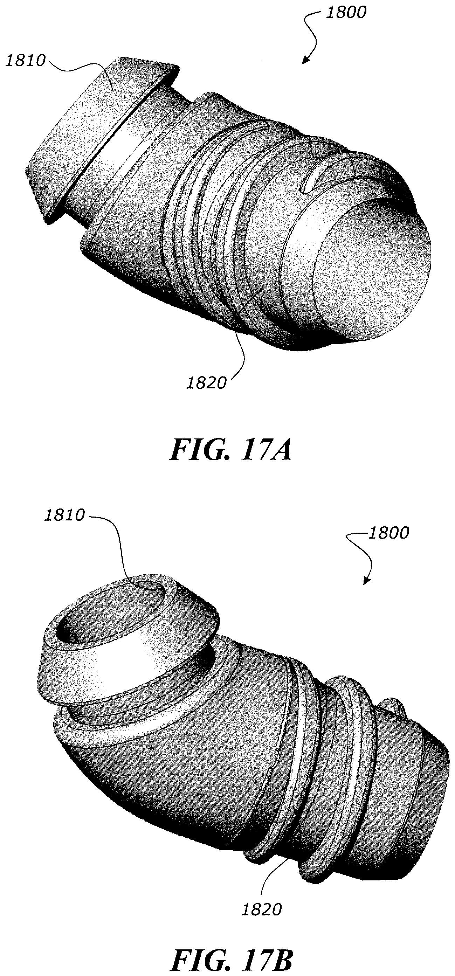

In some embodiments, a swivel connector, such as a swivel elbow, may be attached or overmoulded to the interface tubes to provide a connection (e.g., a curved connection) between the interface tube and a mask and to facilitate adjusting the position of the interface tube relative to the mask. FIGS. 17A-B illustrate such a swivel elbow 1800 separately from an interface tube or a mask. As shown in FIGS. 17A-B, the swivel elbow 1800 may have a barbed end 1810 for being received by a corresponding inner flange of a mask, which can be similar to the internal flange 1343. In some embodiments, the swivel elbow 1800 may have a threaded end 1820 for connection to an interface tube. In some embodiments, other mechanisms for connecting the swivel elbow 1800 and the mask or the interface tube, such as ultrasonic welding or overmolding, may be employed and the swivel elbow may have a suitable structure.

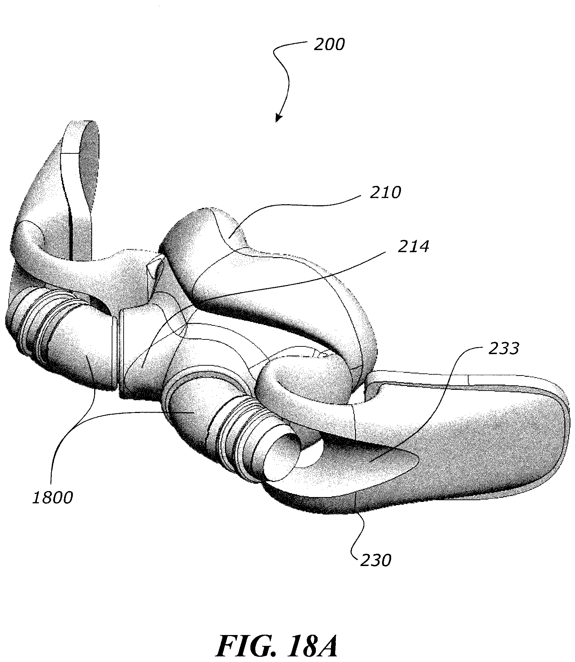

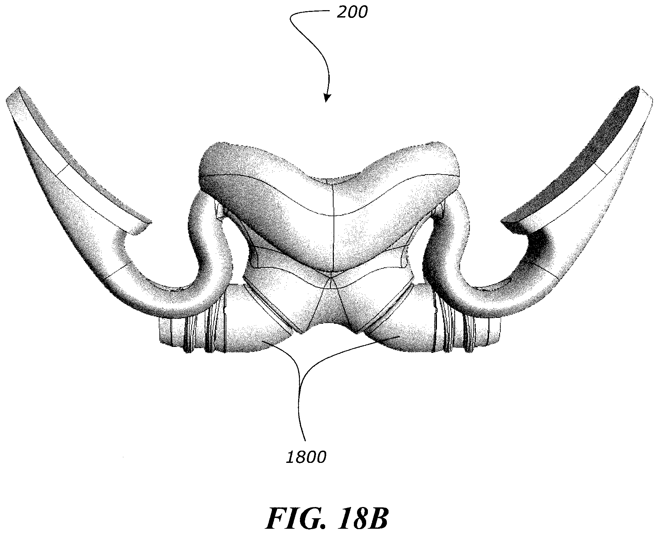

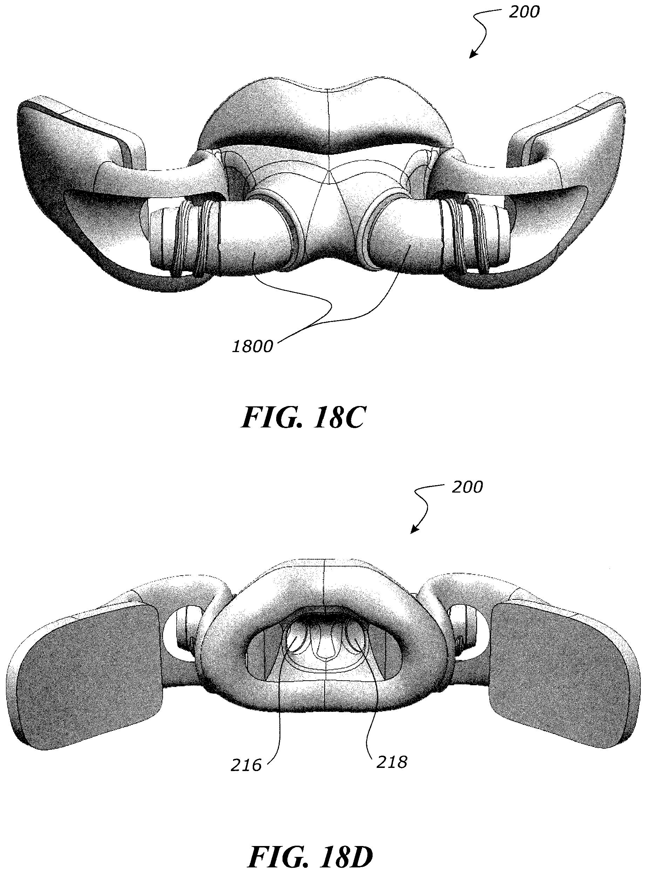

FIGS. 18A-D illustrates the mask 200 with the swivel elbows 1800 attached to the openings 216, 218 of the tube receiving portion 214. The swivel elbow 1800 may provide adjustment of positioning the interface tubes by swiveling around an axis or multiple axes (e.g., a ball-and-socket joint). The swivel elbow 1800 may reduce the bend radius of the interface tubes, thus ease the strain on the interface tubes when the interface tubes are bent and placed along the groove 233 of the bridging portions 230. The swivel elbow 1800 may further allow the interface tubes to be directed up the patient's face (toward the top of their head) and attached to a headgear or bonnet, which may allow for easier side sleeping positions. Also, the swiveling of the swivel elbow 1800 may allow decoupling of the movement of the interface tubes from the mask, and help maintain a seal between the seal body 210 and the user's face when the tube is moved or bumped. The swivel elbow 1800 may be attached to any masks described elsewhere herein, such as the mask 1000, 1300, 1400, 1500, or 1700, to facilitate movement and guidance of the interface tubes.

In some embodiments, the connection between any of the interface tube, the mask, the tube extension, and the swivel elbow described in the specification may be permanent (e.g. overmoulded, attached by adhesive). Such permanent connection may be beneficial as it may withstand a stronger force.

### Other Features of the Bridging Portions

As shown in FIGS. 12A-E and 18A-D, any of the bridging portions described herein may have cutouts and/or grooves on the front/non-patient facing side to guide and accommodate the interface tubes when the interface tubes are placed to the sides, closer to the user's face. In this specification, a term "cutout" means any region of the bridging portions with a hole where all of the material of the bridging portion has been removed. A term "groove" means any region of the bridging portions that has been thinned by a depression in the surface. The size of the cutout, such as the cutout 1734, may be larger than the diameter of the interface tube, such that the interface tube may pass through the cutout. Any bridging portion may have one or more grooves and/or cutouts. Hook or loop material may be positioned within the cutouts and/or grooves. The hook or loop material may attach to corresponding hook or loop material on interface tubes or tube extensions on the mask to aid with securement and positioning of these components. With cutouts and/or grooves, the interface tubes can be placed closer to the user's face, reducing the bulkiness of the patient interface. Further, cutouts and/or grooves may reduce the amount of material used for the bridging portions, thereby reducing costs and reducing the weight of the mask. Also, the cutouts and/or grooves may allow more clearance between the interface tubes and the bridging portions, such that the interface tubes are less likely to contact the bridging portions and interfere with the rolling movement of the bridging portions.

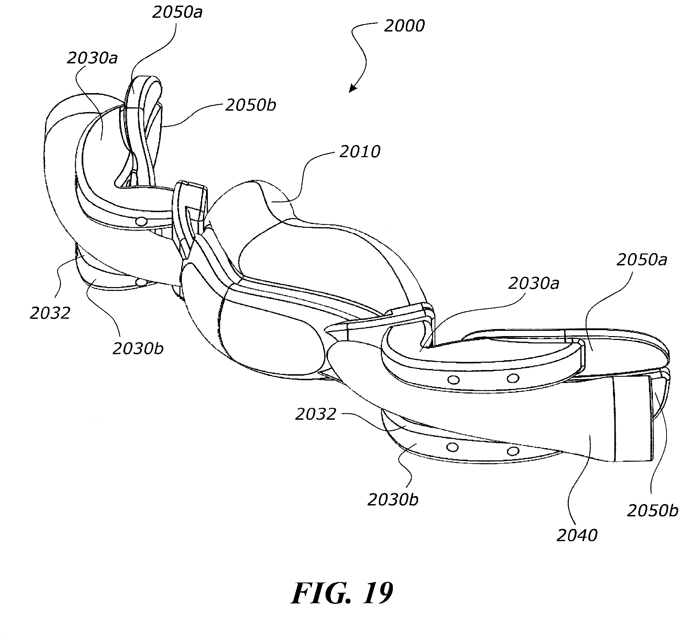

The cutouts or grooves may have any suitable shape, and be placed at any suitable region along the bridging portions and/or the facial pads. In some embodiments, as illustrated in FIG. 19, a mask 2000 may include cutouts 2032 extending along each of entire bridging portions 2030 and facial pads 2050, thereby splitting the assembly of the bridging portion 2030 and the facial pad 2050 into an upper part 2030a, 2050a and a lower part 2030b, 2050b. Such split of the bridging portion and the facial pad may allow independent movement of the upper part and the lower part, and independent adjustment of the upper part and the lower part. For example, if the mask is leaking gas into the user's eye region, the user or a clinician may adjust the top part. The upper part and the lower part of the bridging portion may independently move by rolling as described elsewhere herein. Additionally, such arrangement may provide greater stability as a result of spacing the upper part and lower part vertically from one another, thereby reducing rotation of the mask 2000 about the user's face. The mask 2000 may further include tube extensions 2040 which extend from a seal body 2010 and are positioned between the upper parts and the lower parts and can receive the interface tubes.

FIG. 20 illustrates a mask 2100 similar to the mask 2000. The mask 2100 may include bridging portions 2130 and facial pads 2150 split into upper parts 2130a, 2150a and lower parts 2130b, 2130b. The bridging portions 2130a, 2130b may extend from a center portion of a seal body 2110, and the two bridging portions 2130a, 2130b may adjacent one another without a tube extension located in between. In the illustrated arrangement, the bridging portions 2130 contact one another or are connected along a portion 2190. Such an arrangement can provide additional stability to the combined bridging portions 2130. In the illustrated arrangement, the split upper and lower parts 2130a, 2130b of the bridging portions 2130 are located between the contacting or connected portion 2190 and the respective facial pads 2150.

In some embodiments, each of the bridging portions 2130 includes or defines a substantially U-shaped or C-shaped portion as viewed from above. Preferably, the bridging portions 2130 are configured to provide similar functionality and one or more of the advantages of the other bridging portions described herein. For example, the bridging portions 2130 can generate a sealing force on the seal body 2110 in use. The bridging portions 2130 can provide at least some de-coupling of the facial pads 2150 from the seal body 2110. Each of the bridging portions 2130 can define a path length or an effective length that is greater than a (e.g., lateral or linear) distance between the seal body 2110 and the associated facial pad 2150. Such an arrangement can provide the bridging portion 2130 with a relatively flat force-extension curve, as described above.

### Terminology

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to". Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment.

The term "plurality" refers to two or more of an item. Recitations of quantities, dimensions, sizes, formulations, parameters, shapes and other characteristics should be construed as if the term "about" or "approximately" precedes the quantity, dimension, size, formulation, parameter, shape or other characteristic. The terms "about" or "approximately" mean that quantities, dimensions, sizes, formulations, parameters, shapes and other characteristics need not be exact, but may be approximated and/or larger or smaller, as desired, reflecting acceptable tolerances, conversion factors, rounding off, measurement error and the like and other factors known to those of skill in the art. Recitations of quantities, dimensions, sizes, formulations, parameters, shapes and other characteristics should also be construed as if the term "substantially" precedes the quantity, dimension, size, formulation, parameter, shape or other characteristic. The terms "approximately," "about," and "substantially" as used herein represent an amount close to the stated amount that still performs a desired function or achieves a desired result. For example, in some embodiments, as the context may dictate, the terms "approximately", "about", and "substantially" may refer to an amount that is within less than or equal to 10% of the stated amount. The term "generally" as used herein represents a value, amount, or characteristic that predominantly includes, or tends toward, a particular value, amount, or characteristic. For example, as the context may dictate, the term "generally linear" can mean something that departs from exactly parallel by less than or equal to 15°.

Numerical data may be expressed or presented herein in a range format. It is to be understood that such a range format is used merely for convenience and brevity and thus should be interpreted flexibly to include not only the numerical values explicitly recited as the limits of the range, but also interpreted to include all of the individual numerical values or sub-ranges encompassed within that range as if each numerical value and sub-range is explicitly recited. As an illustration, a numerical range of "1 to 5" should be interpreted to include not only the explicitly recited values of about 1 to about 5, but should also be interpreted to also include individual values and sub-ranges within the indicated range. Thus, included in this numerical range are individual values such as 2, 3 and 4 and sub-ranges such as "1 to 3," "2 to 4" and "3 to 5," etc. This same principle applies to ranges reciting only one numerical value (e.g., "greater than 1") and should apply regardless of the breadth of the range or the characteristics being described.

A plurality of items may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. Furthermore, where the terms "and" and "or" are used in conjunction with a list of items, they are to be interpreted broadly, in that any one or more of the listed items may be used alone or in combination with other listed items. The term "alternatively" refers to selection of one of two or more alternatives, and is not intended to limit the selection to only those listed alternatives or to only one of the listed alternatives at a time, unless the context clearly indicates otherwise.

Reference to any prior art in this specification is not, and should not be taken as, an acknowledgement or any form of suggestion that that prior art forms part of the common general knowledge in the field of endeavor in any country in the world.

Where, in the foregoing description reference has been made to integers or components having known equivalents thereof, those integers are herein incorporated as if individually set forth.

It should be noted that various changes and modifications to the presently preferred embodiments described herein will be apparent to those skilled in the art. Such changes and modifications may be made without departing from the scope of the invention and without diminishing its attendant advantages. For instance, various components may be repositioned as desired. It is therefore intended that such changes and modifications be included within the scope of the invention. Moreover, not all of the features, aspects and advantages are necessarily required to practice the present invention. Accordingly, the scope of the present invention is intended to be defined only by the claims that follow.

## Claims

1. A patient interface (200, 1700), comprising:
a mask seal body (210, 1710) defining a breathing chamber and having a user-contacting surface configured to contact a face of the user and surround at least the nares of the user;
a pair of facial pads (1750), each of the facial pads configured to engage the face of a user on opposing sides of the mask seal body (210, 1710); and
a pair of bridging portions (230, 1730), each of the bridging portions connecting the mask seal body (210, 1710) and a respective one of the facial pads (1750);
wherein each of the bridging portions (230, 1730) has a curved shape, and the bridging portions (230, 1730) comprise a first neutral configuration and a second deflected configuration, the bridging portions (230, 1730) being movable within a range of adjustment from the first configuration towards the second configuration, the bridging portions in the second deflected configuration urging the mask seal body (210, 1710) toward the user's face to create or maintain sealing engagement between the user-contacting surface and the user's face,
wherein the bridging portions (230, 1730) comprise one or more cutouts configured to receive an interface tube, thereby splitting each of the bridging portions (230, 1730) into an upper section and a lower section.

2. The patient interface (200, 1700) of Claim 1, wherein the bridging portions (230, 1730) comprise a portion having a mechanical fastener component.

3. The patient interface (200, 1700) of Claim 2, wherein the mechanical fastener component is a hook or loop material.

4. The patient interface (200, 1700) of any one of Claims 1-3, wherein the upper section and the lower section of each of the bridging portions (230, 1730) are separate from one another.

5. The patient interface (200, 1700) of any one of Claims 1-4, wherein the bridging portions (230, 1730) comprise an S-shape,a U-shape or a C-shape as viewed from above.

6. The patient interface (200, 1700) of any one of Claims 1-5, wherein the bridging portions (230, 1730) comprise one or more hinges.

7. The patient interface (200, 1700) of any one of Claims 1-6, wherein the mask seal body comprises an upper region, wherein at least part of the upper region is configured to roll when the mask seal body is positioned on a user's face.

8. The patient interface (200, 1700) of Claim 7, wherein the upper region comprises a portion with a thinner wall located between portions having thicker walls.

9. The patient interface (200, 1700) of any one of Claims 1-8, further comprising one or more respiratory tubes configured to deliver gas.

10. The patient interface (200, 1700) of Claim 9, wherein the respiratory tubes comprise an inspiratory tube configured to deliver gas to a user and an expiratory tube configured to deliver gas from a user.

11. The patient interface (200, 1700) of any one of Claims 1-10, further comprising a patch assembly coupled to each of the facial pads for securing the facial pads to the user.

12. The patient interface (200, 1700) of Claim 11, wherein the patch assembly comprises a first portion coupled to the facial pad and a second portion configured to be coupled to the user, wherein the patch assembly is configured for releasable connection between the first portion and the second portion.

13. The patient interface (200, 1700) of any one of claims 11 to 12, further comprising a securement system to secure the patient interface to the patient's face, the securement system comprising an adjustable bonnet, wherein the first portion of the patch assembly is connectable to the adjustable bonnet.

14. The patient interface (200, 1700) of any one of Claims 1-13, further comprising a third facial pad configured to engage the forehead of the user above the mask seal body and a third bridging portion connecting the mask seal body and the third facial pad.

## Patentansprüche

1. Patientenverbindungseinrichtung (200, 1700), umfassend:
einen Maskendichtungskörper (210, 1710), der eine Atemkammer definiert und eine Benutzerkontaktoberfläche aufweist, die konfiguriert ist, um mit dem Gesicht des Benutzers in Kontakt zu kommen und mindestens die Nasenlöcher des Benutzers umgibt;
ein Paar Gesichtspolster (1750), wobei jedes Gesichtspolster konfiguriert ist, um das Gesicht eines Benutzers auf gegenüberliegenden Seiten des Maskendichtungskörpers (210, 1710) in Eingriff zu nehmen; und
ein Paar Überbrückungsabschnitte (230, 1730), wobei jeder der Überbrückungsabschnitte den Maskendichtungskörper (210, 1710) und jeweils eines der Gesichtspolster (1750) verbindet;
wobei jeder der Überbrückungsabschnitte (230, 1730) eine gekrümmte Form aufweist und die Überbrückungsabschnitte (230, 1730) eine erste neutrale Konfiguration und eine zweite abgelenkte Konfiguration umfassen, wobei die Überbrückungsabschnitte (230, 1730) innerhalb eines Einstellbereichs von der ersten Konfiguration zu der zweiten Konfiguration hin bewegbar sind, wobei die Überbrückungsabschnitte in der zweiten abgelenkten Konfiguration den Maskendichtungskörper (210, 1710) zu dem des Gesichts des Benutzers hin drängen, um eine abdichtenden Eingriff zwischen der Benutzerkontaktoberfläche und dem Gesicht des Benutzers herzustellen oder aufrechtzuerhalten,
wobei die Überbrückungsabschnitte (230, 1730) einen oder mehrere Ausschnitte umfassen, die konfiguriert sind, um ein Verbindungseinrichtungsrohr aufzunehmen, wodurch jeder der Überbrückungsabschnitte (230, 1730) in einen oberen und einen unteren Teilabschnitt aufgeteilt wird.

2. Patientenverbindungseinrichtung (200, 1700) nach Anspruch 1, wobei die Überbrückungsabschnitte (230, 1730) einen Abschnitt umfassen, der eine mechanische Befestigungskomponente aufweist.

3. Patientenverbindungseinrichtung (200, 1700) nach Anspruch 2, wobei die mechanische Befestigungskomponente ein Haken- oder Schlaufenmaterial ist.

4. Patientenverbindungseinrichtung (200, 1700) nach einem der Ansprüche 1 bis 3, wobei der obere Teilabschnitt und der untere Teilabschnitt von jedem der Überbrückungsabschnitte (230, 1730) voneinander getrennt sind.

5. Patientenverbindungseinrichtung (200, 1700) nach einem der Ansprüche 1 bis 4, wobei die Überbrückungsabschnitte (230, 1730) von oben betrachtet eine S-Form, eine U-Form oder eine C-Form umfassen.

6. Patientenverbindungseinrichtung (200, 1700) nach einem der Ansprüche 1 bis 5, wobei die Überbrückungsabschnitte (230, 1730) ein oder mehrere Scharniere umfassen.

7. Patientenverbindungseinrichtung (200, 1700) nach einem der Ansprüche 1 bis 6, wobei der Maskendichtungskörper eine obere Region umfasst, wobei mindestens ein Teil der oberen Region konfiguriert ist, um zu rollen, wenn der Maskendichtungskörper auf dem Gesicht eines Benutzers positioniert ist.

8. Patientenverbindungseinrichtung (200, 1700) nach Anspruch 7, wobei die obere Region einen Abschnitt mit einer dünneren Wand umfasst, der sich zwischen Abschnitten, die dickere Wände aufweisen, befindet.

9. Patientenverbindungseinrichtung (200, 1700) nach einem der Ansprüche 1 bis 8, ferner umfassend einen oder mehrere Atemschläuche, die konfiguriert sind, um Gas abzugeben.

10. Patientenverbindungseinrichtung (200, 1700) nach Anspruch 9, wobei die Atemschläuche einen Inspirationsschlauch, der konfiguriert ist, um einem Benutzer Gas zuzuführen, und einen Exspirationsschlauch, der konfiguriert ist, um Gas von einem Benutzer abzuführen, umfassen.

11. Patientenverbindungseinrichtung (200, 1700) nach einem der Ansprüche 1 bis 10, ferner umfassend eine Pflasteranordnung, die mit jedem der Gesichtspolster zum Sichern der Gesichtspolster an dem Benutzer gekoppelt ist.

12. Patientenverbindungseinrichtung (200, 1700) nach Anspruch 11, wobei die Pflasteranordnung einen ersten Abschnitt, der mit dem Gesichtspolster gekoppelt ist, und einen zweiten Abschnitt, der konfiguriert ist, um mit dem Benutzer gekoppelt zu werden, umfasst, wobei die Pflasteranordnung für eine lösbare Verbindung zwischen dem ersten Abschnitt und dem zweiten Abschnitt konfiguriert ist.

13. Patientenverbindungseinrichtung (200, 1700) nach einem der Ansprüche 11 bis 12, ferner umfassend ein Sicherungssystem, um die Patientenverbindungseinrichtung an dem Gesicht des Patienten zu sichern, das Sicherungssystem umfassend eine einstellbare Haube, wobei der erste Abschnitt der Pflasteranordnung mit der einstellbaren Haube verbindbar ist.

14. Patientenverbindungseinrichtung (200, 1700) nach einem der Ansprüche 1 bis 13, ferner umfassend ein drittes Gesichtspolster, das konfiguriert ist, um die Stirn des Benutzers oberhalb des Maskendichtungskörpers in Eingriff zu nehmen, und einen dritten Überbrückungsabschnitt, der den Maskendichtungskörper und das dritte Gesichtspolster verbindet.

## Revendications

1. Interface patient (200, 1700), comprenant :
un corps de joint de masque (210, 1710) définissant une chambre de respiration et ayant une surface de contact utilisateur conçue pour venir en contact avec un visage de l'utilisateur et entourer au moins les narines de l'utilisateur ;
une paire de coussinets de visage (1750), chacun des coussinets de visage étant conçu pour venir en prise avec le visage d'un utilisateur sur des côtés opposés du corps de joint de masque (210, 1710) ; et
une paire de portions de pontage (230, 1730), chacune des portions de pontage reliant le corps de joint de masque (210, 1710) et un respectif parmi les coussinets de visage (1750) ;
dans laquelle chacune des portions de pontage (230, 1730) a une forme incurvée, et les portions de pontage (230, 1730) comprennent une première configuration neutre et une seconde configuration fléchie, les portions de pontage (230, 1730) étant mobiles à l'intérieur d'une plage d'ajustement allant de la première configuration à la seconde configuration, les portions de pontage dans la seconde configuration fléchie pressant le corps de joint de masque (210, 1710) en direction du visage de l'utilisateur pour créer ou maintenir une mise en prise étanche entre la surface de contact utilisateur et le visage de l'utilisateur,
dans laquelle les portions de pontage (230, 1730) comprennent une ou plusieurs découpes conçues pour recevoir un tube d'interface, divisant de ce fait chacune des portions de pontage (230, 1730) en une section supérieure et une section inférieure.

2. Interface patient (200, 1700) selon la revendication 1, dans laquelle les portions de pontage (230, 1730) comprennent une portion ayant un composant de fixation mécanique.

3. Interface patient (200, 1700) selon la revendication 2, dans laquelle le composant de fixation mécanique est un matériau de crochet ou de boucle.

4. Interface patient (200, 1700) selon l'une quelconque des revendications 1 à 3, dans laquelle la section supérieure et la section inférieure de chacune des portions de pontage (230, 1730) sont distinctes l'une de l'autre.

5. Interface patient (200, 1700) selon l'une quelconque des revendications 1 à 4, dans laquelle les portions de pontage (230, 1730) comprennent une forme de S, une forme de U ou une forme de C en vue de dessus.

6. Interface patient (200, 1700) selon l'une quelconque des revendications 1 à 5, dans laquelle les portions de pontage (230, 1730) comprennent une ou plusieurs charnières.

7. Interface patient (200, 1700) selon l'une quelconque des revendications 1 à 6, dans laquelle le corps de joint de masque comprend une région supérieure, dans laquelle au moins une partie de la région supérieure est conçue pour rouler lorsque le corps de joint de masque est positionné sur le visage d'un utilisateur.

8. Interface patient (200, 1700) selon la revendication 7, dans laquelle la région supérieure comprend une portion avec paroi plus mince localisée entre des portions ayant des parois plus épaisses.

9. Interface patient (200, 1700) selon l'une quelconque des revendications 1 à 8, comprenant en outre un ou plusieurs tubes respiratoires conçus pour délivrer du gaz.

10. Interface patient (200, 1700) selon la revendication 9, dans laquelle les tubes respiratoires comprennent un tube inspiratoire conçu pour délivrer du gaz à un utilisateur et un tube expiratoire conçu pour délivrer du gaz en provenance d'un utilisateur.

11. Interface patient (200, 1700) selon l'une quelconque des revendications 1 à 10, comprenant en outre un ensemble timbre accouplé à chacun des coussinets de visage, permettant de fixer les coussinets de visage sur l'utilisateur.

12. Interface patient (200, 1700) selon la revendication 11, dans laquelle l'ensemble timbre comprend une première portion accouplée au coussinet de visage et une seconde portion conçue pour être accouplée à l'utilisateur, dans laquelle l'ensemble timbre est conçu pour une liaison libérable entre la première portion et la seconde portion.

13. Interface patient (200, 1700) selon l'une quelconque des revendications 11 à 12, comprenant en outre un système de fixation pour fixer l'interface patient au visage du patient, le système de fixation comprenant un bonnet ajustable, dans laquelle la première portion de l'ensemble timbre peut être reliée au bonnet ajustable.

14. Interface patient (200, 1700) selon l'une quelconque des revendications 1 à 13, comprenant en outre un troisième coussinet de visage conçu pour venir en prise avec le front de l'utilisateur au-dessus du corps de joint de masque et une troisième portion de pontage reliant le corps de joint de masque et le troisième coussinet de visage.
